# EUROPEAN PATENT SPECIFICATION

(11) **EP 3 875 141 B1**
(45) Date of publication and mention of the grant of the patent: **14.12.2022**
(21) Application number: 19879611.2
(22) Date of filing: 30.10.2019
(51) Int. Cl.: A61M 37/00

(54) **MICRO-NEEDLE ARRAY DEVICE**
MIKRONADELFELDVORRICHTUNG
DISPOSITIF À RANGÉE DE MICRO-AIGUILLES

(30) Priority: 31.10.2018 JP 2018204829
(43) Date of publication of application: 08.09.2021
(73) Proprietor: FUJIFILM Corporation, Tokyo 106-8620 (JP)
(72) Inventor: YOSHIDA Junya, Ashigarakami-gun, Kanagawa 258-8577 (JP); KABATA Koki, Ashigarakami-gun, Kanagawa 258-8577 (JP); KOBAYASHI Yuka, Ashigarakami-gun, Kanagawa 258-8577 (JP)
(74) Representative: Klunker IP Patentanwälte PartG mbB
(86) International application number: PCT/JP2019/042461
(87) International publication number: WO 2020/090845

(56) References cited:
- WO-A1-2016/129184
- JP-A- S46 895
- JP-A- 2008 520 369
- JP-A- 2015 134 288
- JP-A- 2018 191 783
- US-A1- 2012 184 916
- US-A1- 2013 006 187
- US-A1- 2013 096 532
- US-A1- 2016 325 081
- US-A1- 2016 354 589

## Description

### Field of the Invention

The present invention relates to a micro-needle array device that comprises a micro-needle array having a sheet part and a plurality of needle parts; and a pressing member for pressing a surface of the sheet part, the surface being on an opposite side of the surface having the needle parts.

### Description of the Related Art

Regarding a method for administering a drug through the surface of a living body, that is, the skin, the mucous membrane, and the like, methods of attaching a liquid substance or a powdery substance have been conventionally known. However, since the attachment regions for these substances are limited to the surface of the skin, the attached drugs may be removed by sweating or contact with foreign materials, and it has been difficult to administer an appropriate amount of a drug. In addition, in such a method of utilizing permeation by diffusion of a drug, absorption of the drug is blocked by the barrier layer of keratin, and it has been difficult to obtain satisfactory drug efficacy. Particularly, with regard to biopharmaceuticals, which have been attracting attention in recent years, since it is very difficult for them to break through the barrier layer by permeation, administration by injection has been selected. However, administration by injection requires the skills of medical workers and is associated with pain and a risk of infection. From such a background, attention has been paid to a method of injecting a drug into the skin without pain by using a micro-needle array in which micro-needles (needle-shaped protrusions) having a high aspect ratio and containing a drug are formed, and having the micro-needles to penetrate the keratinous barrier layer.

Patent Document 1 describes a micro-needle chip including a support part and a needle array region formed by providing a plurality of needles on the surface of the support part, the support part having a curved surface shape curved from the back surface toward the surface where the needle array region is formed.

Patent Document 2 describes a spring-type applicator for tapping and sticking micro-needles to the skin, the applicator having an ingot-shaped holding member as a support base for installing the micro-needles, characterized in that springs are installed so as to push out the holding member.

Patent Document 3 describes an applicator for micro-needles for tapping the skin with micro-needles, characterized by having an auxiliary tool as a support base for installing the micro-needles and a tape. It is described in the Examples of Patent Document 3 that a plunger (diameter 5 mm) is installed in a load cell, a PP plate (0.8 mm thick) having a diameter of 12 mm is attached to the tip of the plunger, and micro-needles made of PGA (diameter 10 mm, 2 mm thick, 98 pieces) are further installed at the tip of the PP plate (paragraph 0023 and Fig. 2 of Patent Document 3).

US 2016/0354589 A1 and JP2015-134288 A also disclose applicators for micro-needle arrays.

### Prior Art Documents

### Patent Documents

Patent Document 1: JP6074889B
Patent Document 2: JP2013-027492A
Patent Document 3: WO2011/089907A

### SUMMARY OF THE INVENTION

The present invention provides a micro-needle array device according to claim 1. Further aspects and preferred embodiments are defined in the dependent claims. Aspects, embodiments, examples, and methods of the present disclosure that do not fall within the scope of the appended claims do not form part of the invention and are merely provided for illustrative purposes.

With regard to the micro-needle chip described in Patent Document 1, since the support part of the needles has a curved surface shape, there is a problem that it is difficult for the needles at the lower part of the curved surface to pierce, and since there is a step of curving the surface of the support part after inspection, there is also a problem that an inspection of the needle shape in the final state cannot be carried out. In the spring-type applicator described in Patent Document 2, the pressing part of the micro-needle array has a planar shape having a larger area than the needle part of the micro-needle array. With regard to the configuration of Patent Document 2, there is a problem that force is concentrated on the outer periphery of the pressing part, the portion to which the strongest force is applied deviates from the needle region, and therefore, needles cannot be tapped sufficiently into the skin. Patent Document 3 describes an example in which the area of the pressing face of the pressing member is smaller than the area of the region where the needle part of the micro-needle array is present; however, the influence exerted by the relationship between the area of the pressing face and the area of the region where the needle part is present, on the solubility of the needle part is not clearly understood.

Micro-needle arrays have been attracting attention in recent years as an alternative to administration by injection; however, it has been difficult to obtain drug efficacy that is equal to that of injection. Particularly, in the case of large animals assuming human beings, the epidermis is thick as compared to that of small animals such as mice and rats, it is difficult to tap the needle part of the micro-needle array sufficiently deeply into the skin, and the needles cannot be sufficiently dissolved, so that sufficient drug efficacy cannot be obtained. An object of the present invention is to provide a micro-needle array device that can have the needle part sufficiently dissolved.

The inventors of the present invention conducted a thorough investigation in order to address the above-described problems, and as a result, the inventors found that needles can be sufficiently dissolved by adjusting the ratio of an area A of a pressing face of a pressing member and an area B of a region where a needle part of a micro-needle array is present to be within a predetermined range, and adjusting the thickness of a sheet part to be within a predetermined range, thus completing the invention.

That is, according to the present invention, the following inventions are provided.
(1) A micro-needle array device comprising:
   a micro-needle array having a sheet part and a plurality of needle parts existing on a top face of the sheet part; and
   a pressing member for pressing a surface of the sheet part, the surface being on an opposite side of the surface having the needle parts,
   in which the sheet part has a thickness of 0.1 to 0.6 mm, and
   an area A of a pressing face of the pressing member for pressing the sheet part of the micro-needle array and an area B of a region where the needle parts of the micro-needle array exist, satisfy the formula: 0.3 ≤ A/B ≤ 0.75.
(2) The micro-needle array device according to (1), in which the micro-needle array is formed of a water-soluble polymer.
(3) The micro-needle array device according to (1) or (2), in which the area A of the pressing face is from 30 mm² to 250 mm², and the area B of the region where the needle parts exist is from 100 mm² to 400 mm².
(4) The micro-needle array device according to any one of (1) to (3), in which the pressing face is a flat surface.
(5) The micro-needle array device according to any one of (1) to (4), in which the pressing face is circular-shaped.
(6) The micro-needle array device according to any one of (1) to (4), in which the pressing face is polygonal-shaped.
(7) The micro-needle array device according to any one of (1) to (6), in which the pressing member and the sheet part are adhered to each other.

According to the micro-needle array device of the invention, the needle parts can be sufficiently dissolved.

### BRIEF DESCRIPTION OF THE DRAWINGS

Fig. 1 is a perspective view of a percutaneous absorption sheet having a needle-shaped protrusion.
Fig. 2 is a perspective view of a percutaneous absorption sheet having a needle-shaped protrusion having a different shape.
Fig. 3 is a cross-sectional view of a needle-shaped protrusion of the percutaneous absorption sheet shown in Fig. 1 and Fig. 2.
Fig. 4 is a perspective view of a percutaneous absorption sheet having a needle-shaped protrusion having a different shape.
Fig. 5 is a perspective view of a percutaneous absorption sheet having a needle-shaped protrusion having a different shape.
Fig. 6 is a cross-sectional view of a needle-shaped protrusion of the percutaneous absorption sheet shown in Fig. 4 and Fig. 5.
Figs. 7A, 7B, and 7C are process diagrams of a method for manufacturing a mold.
Figs. 8A, 8B, and 8C are process diagrams of a method for manufacturing a mold having a different shape.
Figs. 9A, 9B, and 9C are process diagrams of a method for manufacturing a mold having a different shape.
Fig. 10 is a partially enlarged view of the mold.
Fig. 11 is a partially enlarged view of the mold.
Fig. 12 is a flow chart of a method for manufacturing a percutaneous absorption sheet.
Figs. 13A, 13B, and 13C are schematic diagrams showing a step of charging a drug solution into needle-shaped depressions of a mold.
Fig. 14 is a perspective view showing a tip of a nozzle.
Fig. 15 is a perspective view showing a tip of another nozzle.
Fig. 16 is a partially enlarged view of a tip of a nozzle and a mold during charging.
Fig. 17 is a partially enlarged view of a tip of a nozzle and a mold during scanning.
Fig. 18 is a schematic configuration diagram of a drug solution charging apparatus.
Fig. 19 is an explanatory diagram showing the relationship between the hydraulic pressure in the nozzle and the supply of a solution including a drug.
Figs. 20A, 20B, 20C, and 20D are schematic diagrams showing a portion of a manufacturing process for a percutaneous absorption sheet.
Figs. 21A and 21B are diagrams illustrating a polymer layer-forming liquid supplying step of a first embodiment.
Figs. 22A and 22B are diagrams illustrating an unpreferable example of the polymer layer-forming liquid supplying step.
Figs. 23A and 23B are diagrams illustrating a method for applying a polymer layer-forming liquid on a mold.
Figs. 24A, 24B, and 24C are diagrams illustrating another method for applying a polymer layer-forming liquid on a mold.
Figs. 25A, 25B, and 25C are diagrams illustrating contraction of the polymer layer-forming liquid according to the shape of the formwork.
Figs. 26A, 26B, and 26C are diagrams illustrating contraction according to the coating shape of the polymer layer-forming liquid.
Figs. 27A, 27B, and 27C are diagrams illustrating contraction of the polymer layer-forming liquid according to another shape of the formwork.
Figs. 28A and 28B are diagrams illustrating the polymer layer-forming liquid supplying step using a formwork having a different shape.
Fig. 29 is a plan view and a cross-sectional view of a precursor plate used in the Examples.
Fig. 30 is a front view showing a state in which a lever member of an applicator is pulled up.
Fig. 31 is a front view showing a state in which the lever member of the applicator is released to punch out a second piston.
Fig. 32 is an exploded perspective view of the applicator.
Fig. 33 is a plan view showing a groove part and a slope of the applicator.
Fig. 34 is a partially enlarged cross-sectional view showing an annular protrusion and a claw part of the applicator.
Fig. 35 is a perspective view showing a claw-shaped member of the applicator.
Fig. 36 is a perspective view of a micro-needle array unit.
Fig. 37 is a cross-sectional view of the micro-needle array unit of Fig. 36 as taken along line I-I.
Fig. 38 is a perspective view of a micro-needle array unit showing a step of tapping a micro-needle array.
Fig. 39 is a diagram showing the relationship between the needle parts (in a case with a needle part diameter of 12.8 mm and a needle part area of 122 mm²) and the pressing part in the Examples.
Fig. 40 is a graph showing the results of calculating a dissolved needle length of a micro-needle array after tapping and dissolution.
Fig. 41 is a graph showing the results of calculating the external / internal ratio of the post-dissolution residual needle length of the micro-needle array after tapping and dissolution.
Fig. 42 is a graph showing the results of calculating the dissolved needle length of the micro-needle array after tapping and dissolution.
Fig. 43 is a graph showing the results of calculating the external / internal ratio of the post-dissolution residual needle length of the micro-needle array after tapping and dissolution.
Fig. 44 is a graph showing the results of calculating the ratio occupied by the administered drug in the total drug content from the dissolved amount and averaging the ratio by the total number of needles.

### DESCRIPTION OF THE PREFERRED EMBODIMENTS

Hereinafter, embodiments of the present invention will be described in detail.

The micro-needle array device of the present invention is a micro-needle array device comprising a micro-needle array having a sheet part and a plurality of needle parts existing on a top face of the sheet part and a pressing member that presses a surface of the sheet part, the surface being on the opposite side of the surface having the needle parts, at the time of tapping the needle parts.

### (Micro-needle array)

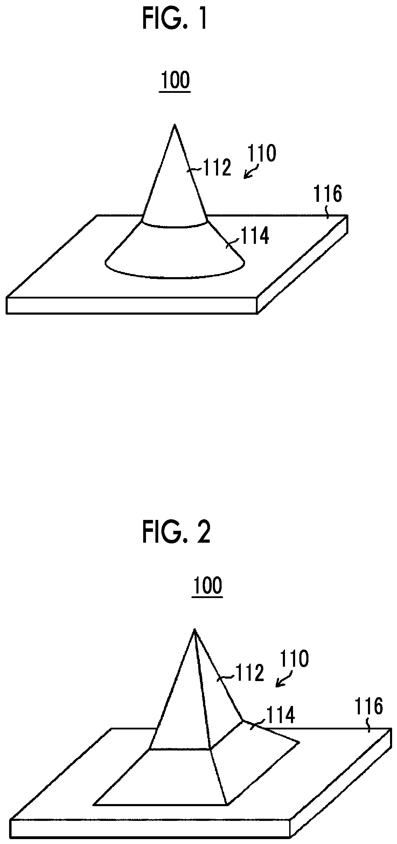

The micro-needle array according to the embodiments of the invention will be described. Fig. 1 and Fig. 2 show a needle-shaped protrusion 110 (also referred to as fine needle or micro-needle), which is a partially enlarged view of the micro-needle array 100.

The micro-needle array 100 supplies a drug into the skin by being stuck to the skin. As shown in Fig. 1, the micro-needle array 100 has a tapered-shaped needle part 112, a frustum part 114 connected to the needle part 112, and a flat plate-shaped sheet part 116 connected to the frustum part 114. The needle-shaped protrusion 110 is composed of the tapered-shaped needle part 112 and the frustum part 114.

A plurality of the frustum parts 114 are formed on the surface of the sheet part 116 (only one frustum part 114 is shown in Fig. 1). An end face having a larger area (lower surface) between the two end faces of the frustum part 114 is connected to the sheet part 116. An end face having a smaller area (upper surface) between the two end faces of the frustum part 114 is connected to the needle part 112. That is, between the two end faces of the frustum part 114, the area of the end face that is in the direction away from the sheet part 116 is decreased. Since the surface with a larger area of the needle part 112 is connected to the end face with a narrower area of the frustum part 114, the needle part 112 has a gradually tapered shape in the direction away from the frustum part 114.

In Fig. 1, the frustum part 114 has a shape of a truncated cone, and the needle part 112 has a shape of a cone. The shape of the tip of the needle part 112 can be appropriately changed to a curved surface having a radius of curvature of from 0.01 µm to 50 µm, a flat surface, or the like, according to the degree of insertion of the needle part 112 into the skin.

Fig. 2 shows a needle-shaped protrusion 110 having a different shape. In Fig. 2, the frustum part 114 has a shape of a truncated quadrangular pyramid, and the needle part 112 has a shape of a quadrangular pyramid.

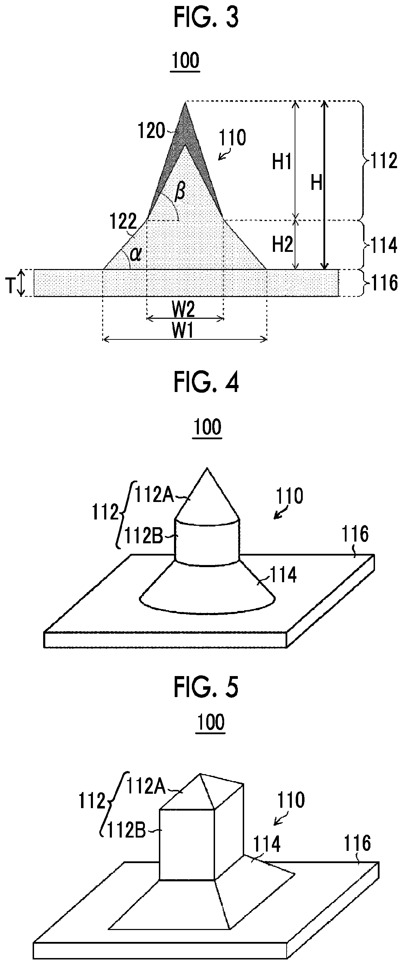

Fig. 3 is a cross-sectional view of the micro-needle array 100 shown in Fig. 1 and Fig. 2. As shown in Fig. 3, the micro-needle array 100 is composed of a drug layer 120 including a predetermined amount of drug and a polymer layer 122. Here, including a predetermined amount of a drug means that a drug in an amount that exhibits drug efficacy at the time of being tapped on the body surface is included. The drug layer 120 including a drug is formed at the tip of the needle-shaped protrusion 110 (tip of the needle part 112). By forming the drug layer 120 at the tip of the needle-shaped protrusion 110, the drug can be efficiently delivered into the skin. Hereinafter, "including a predetermined amount of a drug" will be described as "including a drug", as necessary.

The polymer layer 122 is formed in the portion of the needle part 112 excluding the drug layer 120. The frustum part 114 is composed of the polymer layer 122. The sheet part 116 is composed of the polymer layer 122. The distribution of the drug layer 120 and the polymer layer 122 constituting the needle part 112, the frustum part 114, and the sheet part 116 can be appropriately set.

The thickness T of the sheet part 116 is in the range of from 0.1 mm to 0.6 mm, and preferably in the range of from 0.2 mm to 0.5 mm.

The width W1 of the portion in contact with the frustum part 114 and the sheet part 116 (underside of the frustum part) is in the range of from 100 µm to 1500 µm, and preferably in the range of from 100 µm to 1000 µm. The width W2 of the portion in contact with the frustum part 114 and the needle part 112 (top face of the frustum part) is in the range of from 100 µm to 1500 µm, and preferably in the range of from 100 µm to 1000 µm. It is assumed that the width W1 and the width W2 satisfy the formula: W1 > W2 within the above-described value range.

The height H of the needle-shaped protrusion 110 is in the range of from 100 µm to 2000 µm, and preferably in the range of from 200 µm to 1500 µm. Regarding H1/H2, which is the ratio between the height H1 of the needle part 112 and the height H2 of the frustum part 114, H1/H2 is in the range of from 1 to 10, and preferably in the range of from 1.5 to 8. Furthermore, the height H2 of the frustum part 114 is preferably in the range of from 10 µm to 1000 µm.

The angle α formed by a lateral side of the frustum part 114 and a plane parallel to the surface of the sheet part 116 is in the range of from 10° to 60°, and preferably in the range of from 20° to 50°. The angle β formed by a lateral side of the needle part 112 and the top face of the frustum part 114 is in the range of from 45° to 85°, and preferably in the range of from 60° to 80°.

The angle β is preferably larger than or equal to the angle α. This is because the needle-shaped protrusion 110 can be easily inserted into the skin.

Fig. 4 and Fig. 5 show a needle-shaped protrusion 110 having a different shape. The micro-needle array 100 shown in Fig. 1 and Fig. 4 and the micro-needle array 100 shown in Fig. 2 and Fig. 5 have the same shape of the frustum part 114 and different shapes of the needle part 112. The needle part 112 shown in Fig. 4 and Fig. 5 has a needle-shaped part 112A having a tapered shape and a tubular-shaped body part 112B. The bottom face of the needle-shaped part 112A and an end face of the body part 112B are connected. An end face that is not connected to the needle-shaped part 112A between the end faces of the body part 112B is connected to the top face of the frustum part 114.

The needle-shaped part 112A shown in Fig. 4 has a conical shape, and the body part 112B has a columnar shape. The needle-shaped part 112A shown in Fig. 5 has a shape of a quadrangular pyramid, and the body part 112B has a shape of a quadrangular prism.

Since the needle part 112 has the body part 112B, the needle part 112 has a shape having a constant width in the direction away from the frustum part 114. The needle-shaped part 112A of the needle part 112 has a shape that gradually tapers in a direction away from the body part 112B. The tubular-shaped body part 112B is such that the two end faces facing each other have almost the same area. The needle part 112 has a shape that tapers in a broad way. The shape of the tip of the needle part 112 can be appropriately changed to a curved surface having a radius of curvature of from 0.01 µm to 50 µm, a flat surface, or the like, according to the degree of insertion of the needle part 112 into the skin.

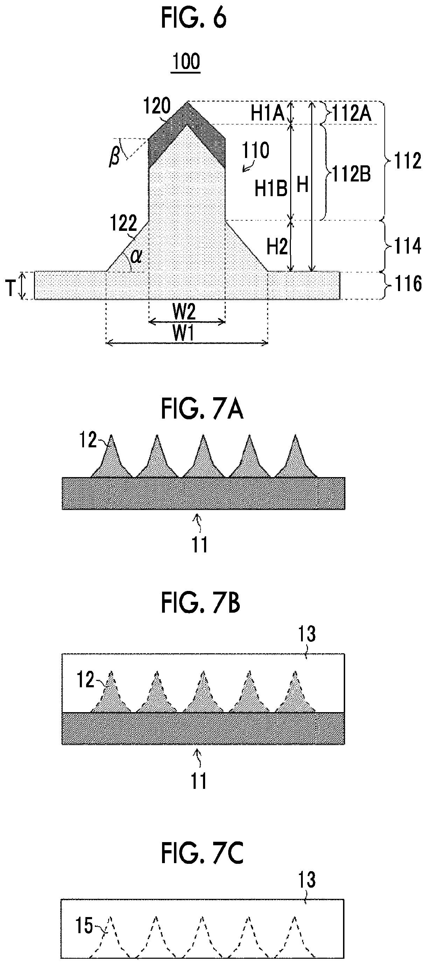

Fig. 6 is a cross-sectional view of the micro-needle array 100 shown in Fig. 4 and Fig. 5. As shown in Fig. 6, the micro-needle array 100 is composed of a drug layer 120 including a drug, and a polymer layer 122. The drug layer 120 including a drug is formed at the tip of the needle-shaped protrusion 110 (tip of the needle part 112). By forming the drug layer 120 at the tip of the needle-shaped protrusion 110, the drug can be efficiently supplied into the skin.

The polymer layer 122 is formed in the portion of the needle part 112 excluding the drug layer 120. The frustum part 114 is composed of the polymer layer 122. The sheet part 116 is composed of the polymer layer 122. The distribution of the drug layer 120 and the polymer layer 122 constituting the needle part 112, the frustum part 114, and the sheet part 116 can be appropriately set.

The thickness T of the sheet part 116, the width W1 of the underside of the frustum part 114, the width W2 of the top face of the frustum part 114, the height H of the needle-shaped protrusion 110, and the height H2 of the frustum part 114 can be adjusted to lengths similar to those of the micro-needle array 100 shown in Fig. 3. The ratio of H1/H2, which is the ratio between the height H1 (H1A + H1B) of the needle part 112 and the height H2 of the frustum part 114, can be adjusted to a ratio similar to that of the micro-needle array 100 shown in Fig. 3.

Regarding the ratio H1B/H1A, which is the ratio between the height H1A of the needle-shaped part 112A and the height H1B of the body part 112B, the ratio H1B/H1A is in the range of from 0.1 to 4, and preferably in the range of from 0.3 to 2.

The angle α formed by a lateral side of the frustum part 114 and a plane parallel to the surface of the sheet part 116 is in the range of from 10° to 60°, and preferably in the range of from 20° to 50°. The angle β formed by a lateral side of the needle-shaped part 112A and a plane parallel to the end faces of the body part 112B is in the range of from 45° to 85°, and preferably in the range of from 60° to 80°.

The angle β is preferably larger than or equal to the angle α. This is because the needle-shaped protrusion 110 can be easily inserted into the skin.

In the present embodiment, the micro-needle array 100 having the needle parts 112 shown in Figs. 1, 2, 4 and 5 is disclosed; however, the micro-needle array 100 is not limited to these shapes.

### (Mold)

Figs. 7A to 7C are process diagrams for the production of a mold.

As shown in Fig.7A, a precursor plate for producing a mold for manufacturing a micro-needle array is first produced.

There are two kinds of the method for producing this precursor plate 11, and the first method involves applying a photoresist on a Si substrate, and then performing exposure and developing. Then, by performing etching by Reactive Ion Etching (RIE) or the like, a plurality of protrusions 12 having the same shape as the needle-shaped protrusions of the micro-needle array is produced in an array form on the surface of the precursor plate 11. In a case where etching such as RIE is performed to form the protrusions 12 on the surface of the precursor plate 11, the protrusions 12 can be formed by performing etching from an oblique direction while rotating the Si substrate.

The second method is a method of producing the plurality of the protrusions 12 in an array form on the surface of a precursor plate 11 by processing a metal substrate such as Ni with a cutting tool such as a diamond cutting tool.

Next, as shown in Fig. 7B, a mold 13 is produced by utilizing the precursor plate 11. For the conventional production of a mold 13, methods based on Ni electroforming and the like are used. Since the precursor plate 11 has protrusions 12 having a conical shape or a pyramid shape (for example, a quadrangular pyramid) with an acute-angled tip, the shape is precisely transferred to the mold 13, and the mold 13 can be detached from the precursor plate 11. Moreover, four methods that allow manufacture at low cost may be considered.

The first method is a method of pouring a silicone resin obtained by adding a curing agent to polydimethylsiloxane (PDMS, for example, SILGARD 184 manufactured by Dow Corning Corporation) into the precursor plate 11, heat-treating the silicone resin at 100°C to cure the resin, and then detaching the mold 13 from the precursor plate 11. The second method is a method of pouring a UV-curable resin that is cured by being irradiated with ultraviolet radiation, into a precursor plate 11, irradiating the resin with ultraviolet radiation in a nitrogen atmosphere, and then detaching the mold 13 from the precursor plate 11. The third method is a method of pouring a plastic resin such as polystyrene or polymethyl methacrylate (PMMA) dissolved in an organic solvent, into a precursor plate 11 coated with a release agent, volatilizing the organic solvent by drying to cure the resin, and then detaching the mold 13 from the precursor plate 11. The fourth method is a method of producing an inversion product by Ni electroforming.

In this manner, a mold 13 on which needle-shaped depressions 15 having an inversion shape of the protrusions 12 of the precursor plate 11 are two-dimensionally arranged is produced. The mold 13 thus produced is shown in Fig. 7C. The mold 13 can be easily produced any number of times by any of the above-described four methods.

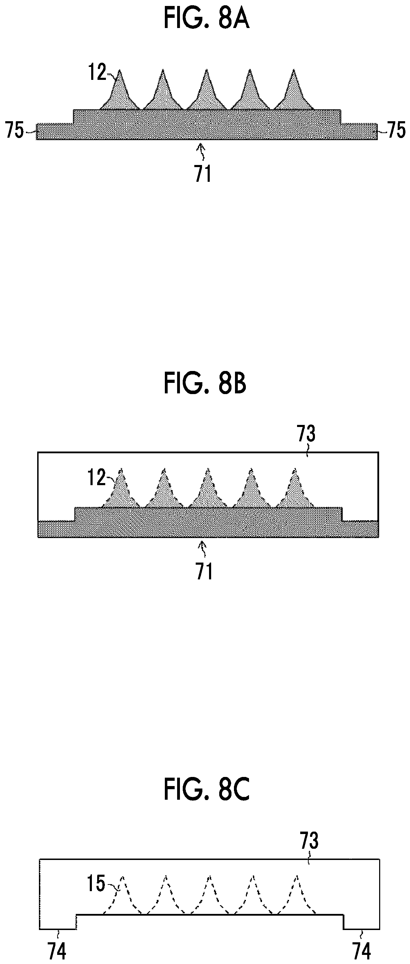

In a case where the mold itself has a level difference part, a mold having an inversion shape can be produced by providing the level difference part on the precursor plate. Figs. 8A to 8C are process diagrams for the production of a mold 73 having, around a region where needle-shaped depressions 15 are formed, a level difference part 74 higher than the region where the needle-shaped depressions 15 are formed.

Similarly to the case of forming a mold that does not have a level difference part, as shown in Fig. 8A, a precursor plate 71 for producing the mold 73 having the level difference part 74 is produced. The precursor plate 71 has formed thereon a level difference part 75 that is lower than the region where the protrusions 12 are formed. The production of a precursor plate can be carried out by a method similar to that shown in Figs. 7A to C.

Next, as shown in Figs. 8B, the mold 73 is produced by utilizing the precursor plate 71. The production of the mold 73 can also be carried out by a method similar to that shown in Fig. 7A to 7C. In this manner, as shown in Fig. 8C, a mold 73 that has the needle-shaped depressions 15 arranged two-dimensionally and has the level difference part 74 around the depressions, which is an inversion shape of the protrusions 12 and the level difference part 75 of the precursor plate 71, is produced.

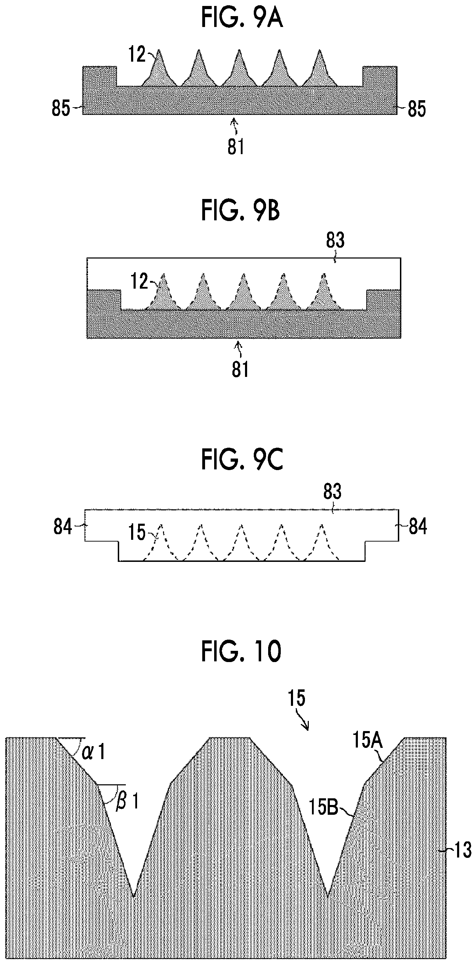

Fig. 9 is a process diagram for the production of a mold 83 having, around a region where needle-shaped depressions 15 are formed, a level difference part 84 lower than the region where the needle-shaped depressions 15 are formed.

Similar to Fig. 7A to Fig. 8C, as shown in Fig. 9A, a precursor plate 81 for producing a mold 83 having a level difference part 84 is produced. The precursor plate 81 has formed thereon a level difference part 85 higher than a region where protrusions 12 are formed.

Next, as shown in Fig. 9B, a mold 83 is produced using the precursor plate 81. In this manner, as shown in Fig. 9C, a mold 83 that has the needle-shaped depressions 15 arranged two-dimensionally and has the level difference part 85 around the depressions, which is an inversion shape of the protrusions 12 and the level difference part 85 of the precursor plate 81, is produced. The method for producing a precursor plate and the method for producing a mold can be carried out by methods similar to those shown in Fig. 7A to Fig. 8C.

Fig. 10 is a partially enlarged view of the needle-shaped depressions 15 of the mold 13. The needle-shaped depressions 15 of the molds 73 and 83 have similar configurations. A needle-shaped depression 15 includes a tapered inlet part 15A that is narrowed in the depth direction from the surface of the mold 13; and a tip depression 15B that tapers in the depth direction. The taper angle α1 of the inlet part 15A basically coincides with the angle α formed by a lateral side of the frustum part and the sheet part of the micro-needle array. Furthermore, the taper angle β1 of the tip depression 15B basically coincides with the angle β formed by a lateral side of the needle part and the top face of the frustum part.

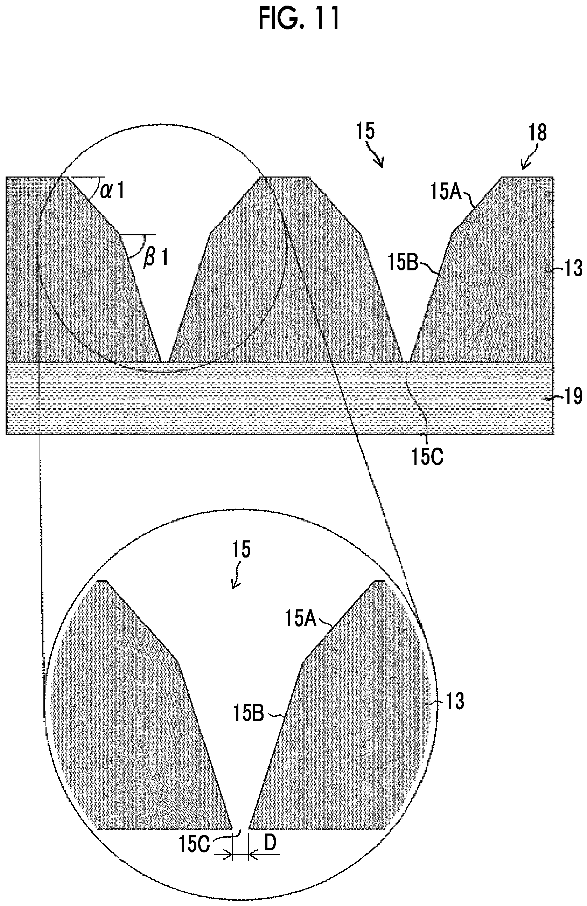

Fig. 11 shows a more preferable embodiment of a mold composite 18 in performing a method for manufacturing a micro-needle array. As shown in Fig. 11, the mold composite 18 is composed of a mold 13 having through-holes 15C formed at the tips of needle-shaped depressions 15; and a gas-permeable sheet 19 that is stuck to the side of the through-holes 15C of the mold 13 and is formed of a material that is permeable to gases but impermeable to liquids. The tip of a needle-shaped depression 15 is in communication with the atmosphere through the gas-permeable sheet 19, by means of a through-hole 15C. The tip of the needle-shaped depression 15 means a side that is tapered in the depth direction of the mold 13, and means a side opposite to the side charged with a drug solution and a polymer layer-forming liquid.

By using such a mold composite 18, the percutaneous absorption material solution charged in the needle-shaped depressions 15 does not permeate, and only the air existing in the needle-shaped depressions 15 can be pulled out through the through-hole 15C from the needle-shaped depressions 15. The transferability at the time of transferring the shape of the needle-shaped depressions 15 to the percutaneous absorption material is improved, and sharper needle-shaped protrusions can be formed.

The size D (diameter) of a through-hole 15C is preferably in the range of 1 to 50 µm. By adjusting the size to this range, air can be easily released, and the tip parts of the needle-shaped protrusions of the micro-needle array can be made into a sharp shape. As the gas-permeable sheet 19 formed of a material that permeates gas but does not permeate liquid, for example, POREFLON (trademark, Sumitomo Electric Industries, Ltd.) can be suitably used.

As the material used for the mold 13, a resin-based material or a metal material can be used. Above all, a resin-based material is preferable, and a material having high gas permeability is more preferable. Oxygen permeability, which is a representative of gas permeability, is preferably higher than 1×10⁻¹² (mL/s-m-Pa), and more preferably higher than 1×10⁻¹⁰ (mL/s·m·Pa). By setting the gas permeability within the above-described range, the air existing in the needle-shaped depressions 15 of the mold 13 can be expelled from the mold 13 side. It is possible to manufacture a micro-needle array having fewer defects. Regarding such a material, as the resin-based material, general engineering plastics such as a silicone resin, an epoxy resin, polyethylene terephthalate (PET), polymethyl methacrylate (PMMA) polystyrene (PS), polyethylene (PE), polyacetal, polyoxymethylene (POM), polytetrafluoroethylene (PTFE), an ultraviolet (UV)-curable resin, a phenolic resin, and a urethane resin can be used. Examples of the metal material include Ni, Cu, Cr, Mo, W, Ir, Tr, Fe, Co, MgO, Ti, Zr, Hf, V, Nb, Ta, α-aluminum oxide, stainless steel and alloys thereof. Furthermore, as will be described later, in the mold 13, since it is necessary to fix the polymer layer-forming liquid at the level difference part in the polymer layer-forming liquid supplying step, it is preferable to use a material having controlled water repellency and wettability. For example, it is preferable that the contact angle between the mold and the polymer layer-forming liquid exceeds 90° and is close to 90°.

### (Polymer solution)

The micro-needle array according to the embodiments of the invention can be formed using a polymer solution, and preferably, the micro-needle array is formed using a water-soluble polymer.

The polymer solution will be described. In the present specification, a polymer solution containing a predetermined amount of a drug is referred to as a polymer solution including a drug or a solution including a drug, as necessary. Furthermore, a polymer solution including a predetermined amount of a drug is referred to as a drug solution. Whether a predetermined amount of drug is included is determined by checking whether the drug can exhibit its efficacy when tapped into the body surface. Therefore, including a predetermined amount of a drug means including an amount of a drug that exhibits drug efficacy when tapped into the body surface.

As the polymer material to be used for the polymer solution, it is preferable to use a water-soluble polymer. Regarding such a water-soluble polymer, sugars such as glucose, maltose, pullulan, chondroitin sulfate, sodium hyaluronate, and hydroxyethyl starch; and proteins such as gelatin can be used. Furthermore, a biodegradable polymer such as polylactic acid or a lactic acid-glycolic acid copolymer may also be used. Among these, a gelatin-based material can be suitably utilized because the gelatin-based material has close adhesiveness to many base materials and also has strong gel strength as a gelling material, so that the material can be closely adhered to a base material in the detaching step that will be described later, and a polymer sheet can be detached from the mold using the base material. Although the concentration varies depending on the material, it is preferable that the concentration is adjusted such that 10% to 50% by mass of the resin polymer is included in the polymer solution forming the polymer layer 122. The solvent to be used for dissolution may be any solvent other than warm water as long as it is volatile, and methyl ethyl ketone (MEK), an alcohol, and the like can be used. Then, in the solution of the polymer resin, it is possible to dissolve all the drugs to be supplied into the body according to the intended uses. The polymer concentration of the polymer solution including the drug forming the drug layer 120 (in a case where the drug itself is a polymer, the concentration of the polymer excluding the drug) is preferably 0% to 30% by mass.

Regarding a method for preparing a polymer solution, in the case of using a water-soluble polymer (gelatin or the like), a water-soluble powder may be dissolved in water, and a drug may be added after the dissolving, or a powder of a water-soluble polymer may be added and dissolved in a liquid in which a drug has been dissolved. In a case where the polymer does not easily dissolve in water, the polymer may be dissolved by heating. The temperature can be appropriately selected depending on the type of the polymer material; however, it is preferable to heat the system at a temperature of about 60°C or lower. It is preferable that the viscosity of the solution of the polymer resin is adjusted preferably to 100 Pa·s or less, and more preferably to 10 Pa·s or less, in the case of a solution including the drug forming the drug layer 120. In the case of a solution forming the polymer layer 122, it is preferable that the viscosity is adjusted preferably to 2000 Pa·s or less, and more preferably to 1000 Pa·s or less. By appropriately adjusting the viscosity of the solution of the polymer resin, the solution can be easily injected into the needle-shaped depressions of the mold. For example, the viscosity of the solution of the polymer resin can be measured with a capillary type viscometer, a falling ball viscometer, a rotational viscometer, or a vibration type viscometer.

### (Drug)

The drug to be incorporated into the polymer solution is not limited as long as it has a function as a drug. In particular, it is preferable to select a drug from peptides, proteins, nucleic acids, polysaccharides, vaccines, pharmaceutical compounds belonging to water-soluble low-molecular weight compounds, or cosmetic ingredients.

### (Method for manufacturing micro-needle array)

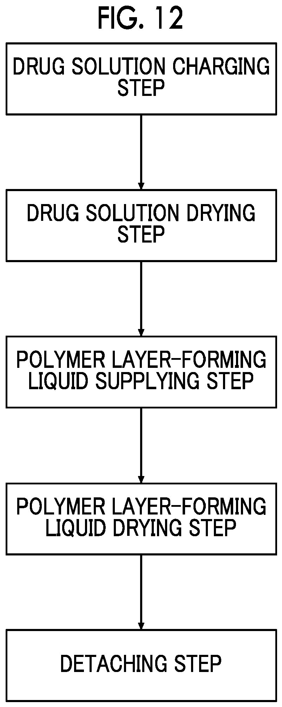

A method for manufacturing a micro-needle array comprises, for example, as shown in Fig. 12, at least five steps, namely, a drug solution charging step, a drug solution drying step, a polymer layer-forming liquid supplying step, a polymer layer-forming liquid drying step, and a detaching step, in this order.

### (Drug solution charging step)

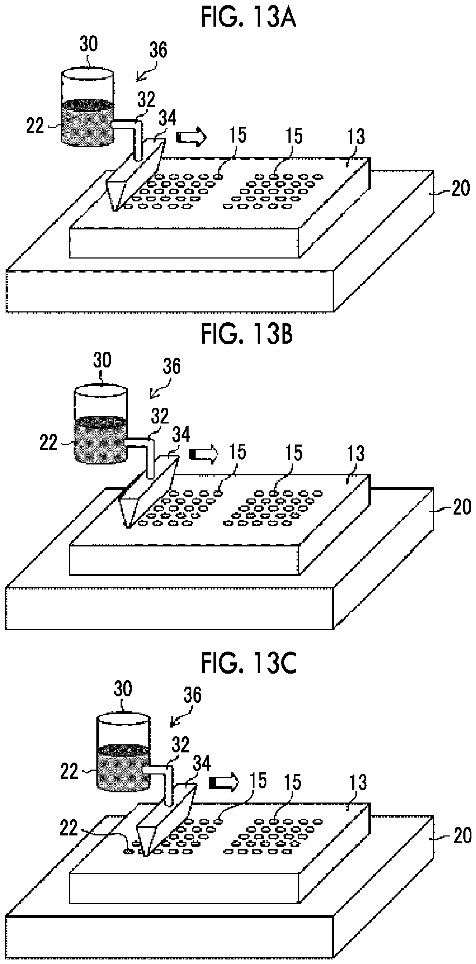

A method of manufacturing a micro-needle array using a mold 13 will be described. As shown in Fig. 13A, the mold 13 having two-dimensionally arranged needle-shaped depressions 15 is disposed on a base 20. The mold 13 has formed thereon two sets of a plurality of the needle-shaped depressions 15 arranged two-dimensionally in a 5×5 array. A liquid supply apparatus 36 having a liquid feeding tank 30 accommodating a drug solution 22, which is a polymer solution including a predetermined amount of a drug; a piping 32 connected to the liquid feeding tank 30; and a nozzle 34 connected to the tip of the piping 32, is prepared. The drug solution 22 is discharged through the tip of the nozzle 34.

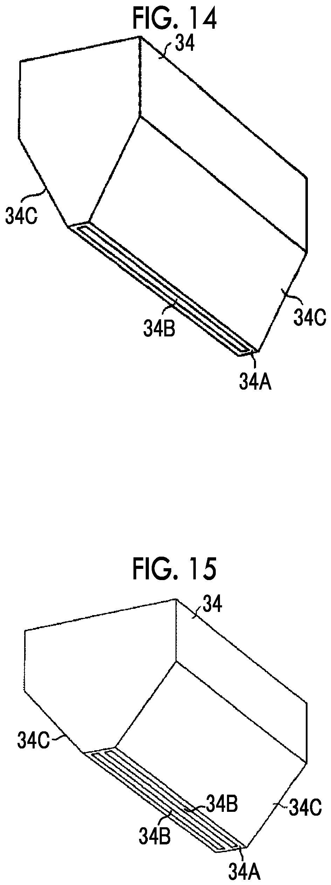

Fig. 14 shows a schematic perspective view of the tip part of the nozzle. As shown in Fig. 14, the nozzle 34 comprises a lip part 34A, which is a flat surface on the tip side; a slit-shaped opening 34B; and two inclined surfaces 34C extending along the lip part 34A and spreading in directions away from the opening 34B. The slit-shaped opening 34B makes it possible to charge a drug solution 22 into, for example, the plurality of the needle-shaped depressions 15 constituting a row at the same time. The size (length and width) of the opening 34B is appropriately selected according to the number of the needle-shaped depressions 15 to be filled at one time.

By increasing the length of the opening 34B, the drug solution 22 can be charged into more needle-shaped depressions 15 at one time. Thereby, productivity can be enhanced.

Fig. 15 shows a schematic perspective view of the tip part of another nozzle. As shown in Fig. 15, the nozzle 34 comprises a lip part 34A, which is a flat surface on the tip side; two slit-shaped openings 34B; and two inclined surfaces 34C extending along the lip part 34A and spreading in directions away from the openings 34B. The two openings 34B make it possible to charge a drug solution 22 including a drug into, for example, the plurality of the needle-shaped depressions 15 constituting two rows at the same time.

As the material used for the nozzle 34, an elastic material or a metal material can be used. For example, TEFLON (registered trademark), stainless steel (SUS), titanium and the like may be mentioned.

The charging step will be described with reference to Fig. 13B. As shown in Fig. 13B, the opening 34B of the nozzle 34 is positioned above the needle-shaped depressions 15. The nozzle 34 for discharging the drug solution 22 is pressed against the mold 13, and the lip part 34A of the nozzle 34 and the surface of the mold 13 are in contact with each other. The drug solution 22 is supplied from the liquid supply apparatus 36 to the mold 13, and the drug solution 22 is charged into the needle-shaped depressions 15 through the opening 34B of the nozzle 34. In the present embodiment, the drug solution 22 is charged into the plurality of the needle-shaped depressions 15 constituting one row at the same time. However, the embodiment is not limited to this, and the drug solution can be charged into the needle-shaped depressions 15 one by one. Furthermore, for the plurality of the needle-shaped depressions 15 constituting a plurality of rows, the drug solution 22 can be charged at the same time into a plurality of rows each time by using the nozzle 34 shown in Fig. 15.

In a case where the mold 13 is formed of a material having gas permeability, the drug solution 22 can be sucked by sucking from the back surface of the mold 13, and the charging of the drug solution 22 into the needle-shaped depressions 15 can be accelerated.

Following the charging step described with reference to Fig. 13B, as shown in Fig. 13C, the liquid supply apparatus 36 is relatively scanned in a direction perpendicular to the length direction of the opening 34B, while the lip part 34A of the nozzle 34 and the surface of the mold 13 are brought into contact with each other. The nozzle 34 is scanned above the mold 13, and the nozzle 34 is moved to the needle-shaped depressions 15 into which the drug solution 22 has not been charged. The opening 34B of the nozzle 34 is positioned above the needle-shaped depressions 15. In the present embodiment, an example of scanning the nozzle 34 has been described; however, the mold 13 may be scanned.

Since the lip part 34A of the nozzle 34 and the surface of the mold 13 are brought into contact with each other, and the nozzle 34 is scanned above the mold 13, the nozzle 34 can scrape off the drug solution 22 remaining on the surface other than the needle-shaped depressions 15 of the mold 13. It is possible to prevent the drug solution 22 including a drug from remaining in areas other than the needle-shaped depressions 15 on the mold 13. Furthermore, in the present embodiment, the nozzle 34 is disposed such that the longitudinal direction of the inclined surfaces 34C orthogonally intersects the scanning direction indicated by the arrow. Therefore, the nozzle 34 can be scanned smoothly above the mold 13.

In order to reduce damage to the mold 13 and to suppress deformation caused by compression of the mold 13 as much as possible, it is preferable to control the degree of pressing of the nozzle 34 against the mold 13 during scanning. For example, it is preferable to control the pressing force of the nozzle 34 against the mold 13 and a push-in distance of the nozzle 34 into the mold 13. In order to prevent the drug solution 22 from remaining in areas other than the needle-shaped depressions 15 on the mold 13, it is desirable that at least one of the mold 13 or the nozzle 34 is a flexible and elastically deformable material.

By repeating the charging step of Fig. 13B and the scanning step of Fig. 13C, the drug solution 22 is charged into the needle-shaped depressions 15 that are two-dimensionally arranged in a 5×5 array. As the drug solution 22 is charged into the needle-shaped depressions 15 that are two-dimensionally arranged in a 5×5 array, the liquid supply apparatus 36 is moved to adjacent needle-shaped depressions 15 that are two-dimensionally arranged in a 5×5 array, and the charging step of Fig. 13B and the scanning step of Fig. 13C are repeated. The drug solution 22 is also charged into the adjacent needle-shaped depressions 15 that are two-dimensionally arranged in a 5×5 array.

Regarding the above-mentioned charging step and scanning step, (1) a mode in which the drug solution 22 is charged into the needle-shaped depressions 15 while scanning the nozzle 34 may be adopted, or (2) a mode in which during scanning of the nozzle 34, the nozzle 34 is temporarily stopped on the needle-shaped depressions 15, the drug solution 22 is charged, and then the nozzle 34 is scanned again after charging, may be adopted. Between the charging step and the scanning step, the lip part 34A of the nozzle 34 is pressed against the surface of the mold 13. The amount of the drug solution 22 discharged from the liquid supply apparatus 36 is preferably adjusted to an amount equal to the total volume of the plurality of the needle-shaped depressions 15 of the mold 13 to be filled. The drug solution 22 can be prevented from remaining on the surface other than the needle-shaped depressions 15 of the mold 13, and the loss of the drug can be reduced.

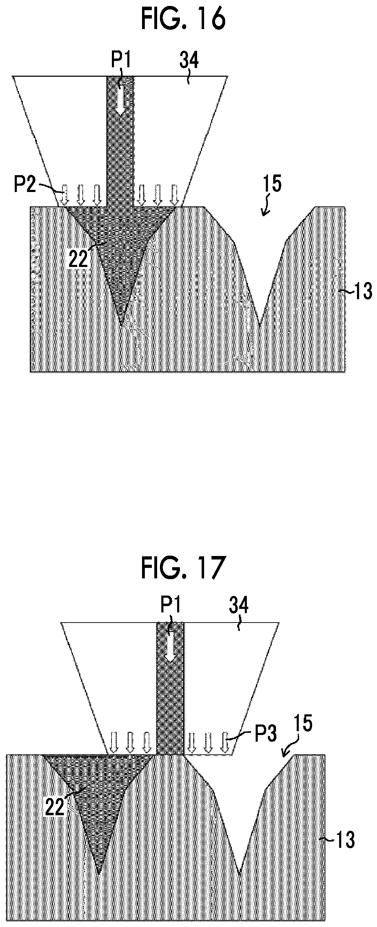

Fig. 16 is a partially enlarged view of the tip of the nozzle 34 and the mold 13 at the time of charging the drug solution 22 into the needle-shaped depressions 15. As shown in Fig. 16, by applying a pressurizing force P1 inside the nozzle 34, it is possible to accelerate the charging of the drug solution 22 into the needle-shaped depressions 15. Moreover, at the time of charging the drug solution 22 into the needle-shaped depressions 15, it is preferable that a pressing force P2 for bringing the nozzle 34 into contact with the surface of the mold 13 is adjusted to be equal to or greater than the pressurizing force P1 inside the nozzle 34. By satisfying the condition: pressing force P2 ≥ pressurizing force PI, it is possible to prevent the drug solution 22 from leaking from the needle-shaped depressions 15 to the surface of the mold 13.

Fig. 17 is a partially enlarged view of the tip of the nozzle 34 and the mold 13 while the nozzle 34 is moving. In a case where the nozzle 34 is scanned relative to the mold 13, it is preferable that a pressing force P3 that brings the nozzle 34 into contact with the surface of the mold 13 is made smaller than the pressing force P2 that brings the nozzle 34 during charging into contact with the surface of the mold 13. This is to reduce damage to the mold 13 and to suppress deformation caused by compression of the mold 13.

It is preferable that the lip part 34A of the nozzle 34 is parallel to the surface of the mold 13. The posture of the nozzle 34 may be controlled by providing a joint driving mechanism at the installation part of the nozzle 34.

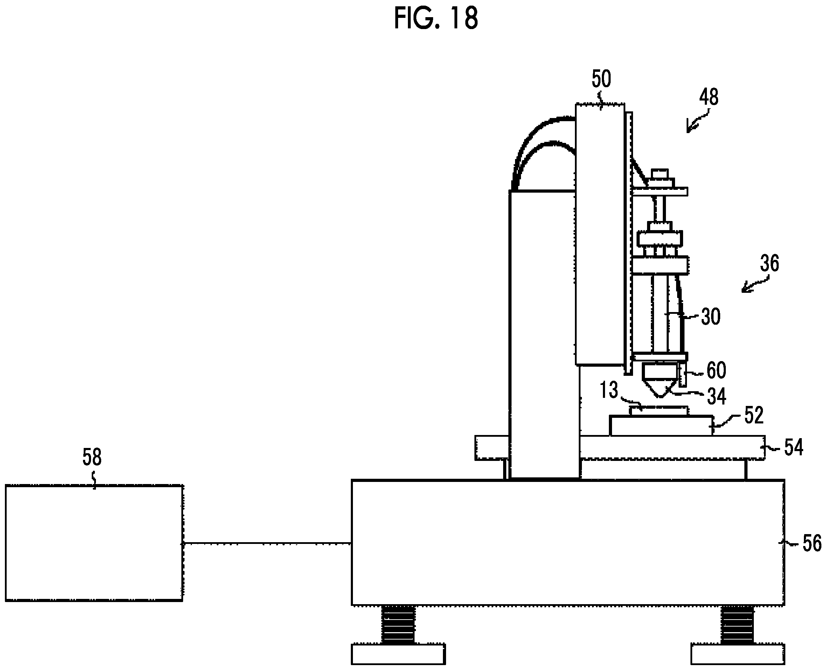

It is preferable to control the pressing force and/or the push-in distance of the nozzle 34 against the mold 13 by driving the nozzle 34 in the Z-axis direction according to the surface shape of the mold 13. Fig. 18 is a schematic configuration diagram of a drug solution charging apparatus 48 capable of controlling the pressing force and/or the push-in distance. The drug solution charging apparatus 48 has a liquid supply apparatus 36 having a liquid feeding tank 30 that accommodates the drug solution and the nozzle 34 attached to the liquid feeding tank 30; and a Z-axis driving unit 50 that drives the liquid feeding tank 30 and the nozzle 34 in the Z-axis direction; a suction base 52 for mounting the mold 13; an X-axis driving unit 54 that drives the suction base 52 in the X-axis direction; a trestle 56 that supports the apparatus; and a control system 58.

A case where the pressing force is controlled to be constant will be described. The nozzle 34 is brought closer to the mold 13 by the Z-axis driving unit 50 to Z-coordinates where a desired pressing force is obtained. While the nozzle 34 that is in contact with the mold 13 is scanned with the X-axis driving unit 54, the drug solution 22 is discharged while controlling the Z-axis coordinates such that the pressing force is constant. The method for measuring the contact pressure is not particularly limited, and for example, various load cells can be used, for example, under the suction base 52 or instead of the suction base 52. A load cell means a measuring instrument capable of measuring a force of compression in the thickness direction. It is preferable that the pressing force can be controlled to be constant at any pressure within the range of 1 to 1000 kPa against the mold 13.

A case where the push-in distance is controlled to be constant will be described. Before bringing the nozzle 34 into contact, the surface shape of the mold 13 is measured in advance. While the nozzle 34 that is in contact with the mold 13 is scanned with the X-axis driving unit 54, the drug solution 22 is discharged while feeding back the value obtained by offsetting the Z-axis coordinates so as to have a desired push-in distance against the surface shape of the mold 13, to the Z-axis driving unit 50.

The method for shape measurement is not particularly limited; however, for example, an optical measuring instrument such as a non-contact type laser displacement meter 60, a contact type stylus type step gauge, and the like can be used. Furthermore, the posture of the nozzle 34 in the slit direction may be controlled according to the surface shape of the mold 13. It is preferable that the push-in distance is controlled in the range of 1% to 15% with respect to the thickness of the mold 13. By operating while controlling the distance between the nozzle 34 and the mold 13 in the Z-axis direction by the Z-axis driving unit 50 according to the shape of the mold 13, the compression deformation rate is made uniform, and the accuracy for the charging amount can be enhanced.

Regarding the control of the pressing force and the push-in distance, in a case where the push-in distance is small, it is preferable to control the pressing force, and in a case where the push-in distance is large, it is preferable to directly control the push-in distance.

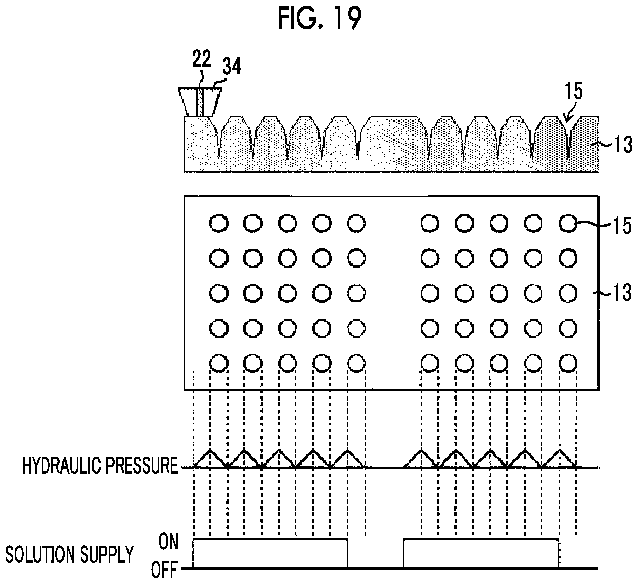

Fig. 19 is an explanatory diagram showing the relationship between the hydraulic pressure in the nozzle and the supply of a solution including a drug. As shown in Fig. 19, the supply of the drug solution 22 is initiated before the nozzle 34 is located over the needle-shaped depressions 15. This is to reliably charge the drug solution 22 into the needle-shaped depressions 15. The drug solution 22 is continuously supplied to the mold 13 until the charging into the plurality of the needle-shaped depressions 15 constituting a 5×5 array is completed. Supplying of the drug solution 22 to the mold 13 is stopped before the nozzle 34 is located above the needle-shaped depressions 15 in the fifth row. It is possible to prevent the drug solution 22 from overflowing from the needle-shaped depressions 15. Regarding the hydraulic pressure in the nozzle 34, as the supply of the drug solution 22 is initiated, the hydraulic pressure increases in a region where the nozzle 34 is not located above the needle-shaped depressions 15. On the other hand, as the nozzle 34 is located above the needle-shaped depressions 15, the drug solution 22 is charged into the needle-shaped depressions 15, and the hydraulic pressure in the nozzle 34 is lowered. Fluctuations in hydraulic pressure are repeated.

As the charging into the plurality of the needle-shaped depressions 15 constituting a 5×5 array is completed, the nozzle 34 is moved to the plurality of the needle-shaped depressions 15 constituting an adjacent 5×5 array. Regarding the liquid supply, it is preferable to stop the supply of the drug solution 22 at the time of moving to the plurality of the needle-shaped depressions 15 constituting an adjacent 5×5 array. There is a distance from the needle-shaped depressions 15 in the fifth row to the needle-shaped depressions 15 in the next first row. In a case where the drug solution 22 is continuously supplied while the nozzle 34 is scanned between the two rows, the hydraulic pressure in the nozzle 34 may be too high. As a result, the drug solution 22 may flow out from the nozzle 34 to an area other than the needle-shaped depressions 15 of the mold 13, and it is preferable to stop the supply of the drug solution 22 in order to suppress this.

At the time of performing charging of the drug solution 22, it is preferable to clean the tip of the nozzle 34 before use. This is because in a case where there is a deposit on the surface of the lip part 34A of the nozzle 34 before charging, the accuracy for the charging amount of the drug solution 22 is deteriorated. Cleaning is generally carried out by wiping with a non-woven fabric. Upon wiping, cleaning can be performed effectively using a non-woven fabric infiltrated with water or a solvent.

At the time of separating the nozzle 34 from the mold 13 after charging of the drug solution 22, there is a possibility that the drug solution 22 may remain on the surface of the mold 13. After the charging into the needle-shaped depressions 15 is completed, the suckback control of sucking the drug solution 22 through the opening 34B of the nozzle 34 can be performed to suck up an excessively discharged portion of the drug solution 22 and reduce the residual liquid on the surface of the mold 13.

In the drug solution charging step, the mold 13 shown in Fig. 11 is utilized, and the drug solution 22 can be charged into the needle-shaped depressions 15 by sucking from the through-hole 15C side.

As the charging of the drug solution 22 into the needle-shaped depressions 15 is completed, the production proceeds to a drug solution drying step, a polymer layer-forming liquid supplying step, a polymer layer-forming liquid drying step, and a detaching step.

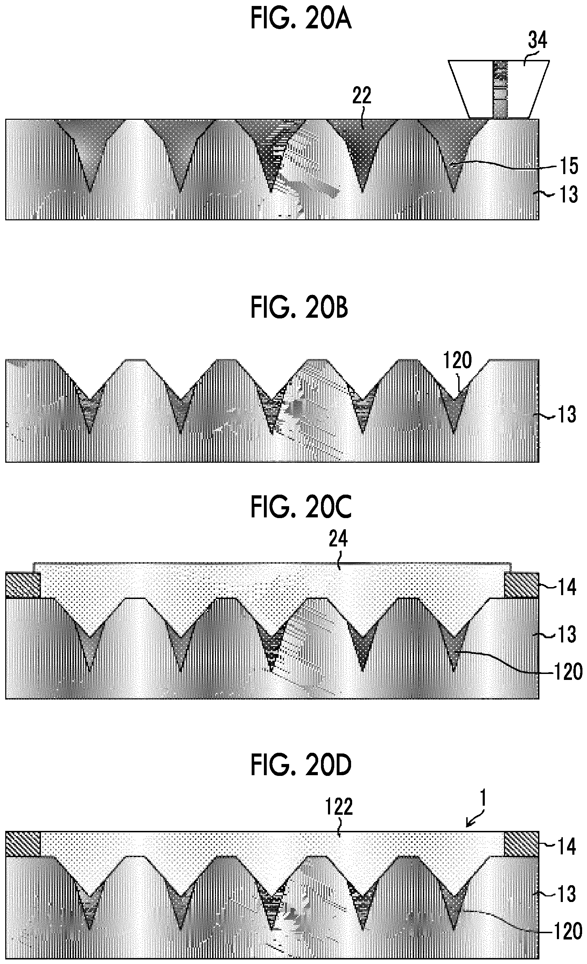

As shown in Fig. 20A, the drug solution 22 is charged into the needle-shaped depressions 15 of the mold 13 through the nozzle 34 in the drug solution charging step. The drug solution charging step is carried out by the above-described method.

### (Drug solution drying step)

As shown in Fig. 20B, in the drug solution drying step, the drug solution 22 is dried and solidified, and thereby the drug layer 120 including the drug is formed in the needle-shaped depressions 15.

The drug solution drying step is a step of drying the drug solution 22 charged in the needle-shaped depressions 15 of the mold 13 and localizing the drug solution at the tips of the needle-shaped depressions 15. It is preferable that the drug solution drying step is carried out in an environment at a temperature of from 1°C to 10°C. By carrying out the step in this range, the occurrence of pore defects can be reduced. Furthermore, by optimizing the drying speed by controlling the temperature and humidity conditions for the drug solution drying step, adhesion of the drug solution 22 to the wall surface of the mold 13 of the needle-shaped depressions 15 can be reduced, and while the drug solution 22 gathers at the tips of the needle-shaped depressions 15, drying proceeds.

It is preferable that drying of the drug solution 22 in the drug solution drying step is performed in a windless state. In a case where the drug solution 22 is directly exposed to non-uniform wind, uneven drying occurs. This is because the drying speed increases at a portion exposed to strong wind, adhesion of the drug solution 22 to the wall surface of the mold 13 occurs, and there is a possibility that the drug solution 22 may be prevented from being localized at the tips of the needle-shaped depressions 15.

In order to realize drying in a windless state, for example, it is preferable to install a windshield. The windshield is installed so that the mold 13 is not directly exposed to wind. As a windshield, a method of installing a physical obstacle such as a lid, a hood, a screen, or an enclosure is preferred due to convenience. At the time of installing the windshield, it is preferable to secure a vent hole or the like so that the installation space for the mold 13 is not in a sealed state. In a case where the installation space is in a sealed state, there is a possibility that water vapor in the sealed space may be saturated, and drying of the drug solution 22 may not proceed. Any vent hole is preferable as long as incoming and outgoing of vapor therethrough is allowed, and in order to stabilize the air flow within the windshield, it is more preferable to cover the vent hole with a water vapor-permeable film or the like. The drying time is appropriately adjusted in consideration of the shape of the needle-shaped depressions 15, the disposition and number of the needle-shaped depressions 15, the type of the drug, the charging amount and concentration of the drug solution 22, and the like.

A windless state means a state in which there is no wind as well as a case where the wind speed is 0.5 m/s or less. This is because uneven drying almost does not occur as the wind speed in this range.

In the drug solution drying step, the drug solution 22 is solidified by drying, and the volume is diminished from the state at the time of charging the drug solution 22. This makes it possible to detach the drug layer 120 easily from the needle-shaped depressions 15 of the mold 13 in the detaching step.

### (Polymer layer-forming liquid supplying step)

Next, as shown in Fig. 20C, a polymer layer-forming liquid 24, which is a polymer solution for forming a polymer layer 122, is supplied on the drug layer 120 including a predetermined amount of the drug, and the polymer layer-forming liquid 24 is charged into the needle-shaped depressions 15. Regarding the supply of the polymer layer-forming liquid, coating by a dispenser, bar coating, spin coating, coating by spraying or the like, and the like can be applied; however, the supply is not limited to these. Hereinafter, an embodiment of supplying the polymer layer-forming liquid 24 to the mold 13 by coating will be described. Since the drug layer 120 including a drug is solidified by drying, it is possible to prevent the drug included in the drug layer 120 from diffusing into the polymer layer-forming liquid 24.

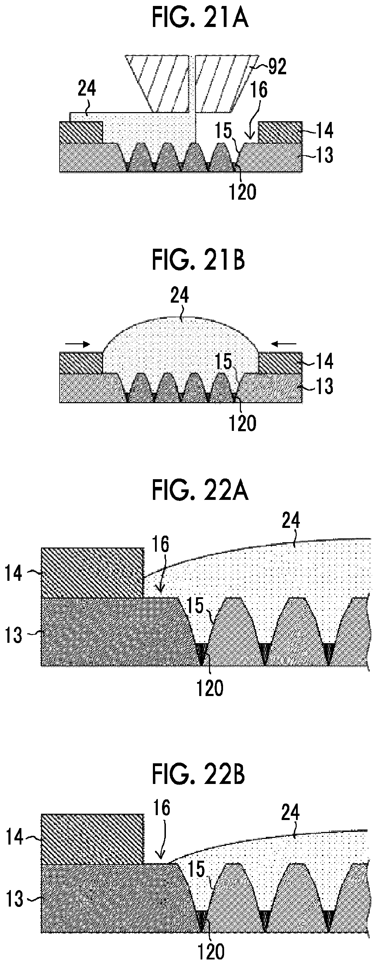

Figs. 21A and 21B are diagrams illustrating a polymer layer-forming liquid supplying step. In the polymer layer-forming liquid supplying step of the first embodiment, a formwork 14 is installed around a region 16 in which the needle-shaped depressions 15 are formed, and the polymer layer-forming liquid 24 is applied on the mold 13 having a level difference part higher than the region 16 where the needle-shaped depressions 15 are formed. The formwork 14 can be installed to be separable from the mold 13.

In the polymer layer-forming liquid supplying step, as shown in Fig. 21A, the polymer layer-forming liquid 24 is applied, using an application unit 92, at a height higher than or equal to that of the level difference part formed by the formwork 14 installed around the needle-shaped depressions 15, and in an area larger than or equal to that of the level difference part as viewed from the top face. Applying the polymer layer-forming liquid to a height higher than or equal to the formwork 14 means the height of the polymer layer-forming liquid 24 at a portion where the polymer layer-forming liquid 24 and the formwork 14 are in contact with each other. In order to make it easy to detach the manufactured micro-needle array, the formwork 14 is formed of a material that easily repels the polymer layer-forming liquid, and after application of the polymer layer-forming liquid 24, the polymer layer-forming liquid 24 is repelled by the formwork 14 and contracts due to surface tension. As shown in Fig. 21B, the contact position between the contracted polymer layer-forming liquid 24 and the mold 13 is fixed at the level difference part of the formwork 14. By drying the polymer layer-forming liquid in a state of being fixed at the formwork 14, the shape of the polymer layer 122 of the micro-needle array (shape of the sheet part 116) can be stably formed.

Figs. 22A and 22B are diagrams illustrating an unpreferable example of the polymer layer-forming liquid supplying step. As shown in Fig. 22A, the polymer layer-forming liquid 24 is applied inside the formwork 14 at a height lower than that of the formwork 14. After application, as shown in Fig. 22B, the polymer layer-forming liquid 24 contracts in the region 16 where the needle-shaped depressions 15 are formed, due to surface tension. In Fig. 22B, since the volume of the polymer layer-forming liquid 24 further contracts due to the polymer layer-forming liquid drying step, which is subsequent to the polymer layer-forming liquid supplying step, there occurs a portion in which the sheet part 116 of the micro-needle array is not stably formed.

As the material for the formwork 14 provided in the mold 13, the same material as that of the above-mentioned mold can be used. Furthermore, as the wettability with the polymer layer-forming liquid is better, the liquid level during drying can be made more uniform and gentler, and any local change in the liquid level shape of the polymer layer-forming liquid can be prevented. In the present invention, as the polymer layer-forming liquid is repelled by the formwork 14 and contracts, the polymer layer-forming liquid is fixed at the level difference part, and therefore, the material for forming the formwork needs to have water repellency against the polymer layer-forming liquid. Therefore, the material for forming the formwork is preferably a material having controlled water repellency and wettability against the polymer layer-forming liquid, and the contact angle of the formwork with respect to the polymer layer-forming liquid is preferably more than 90° and close to 90°. By using a material having satisfactory wettability with the polymer layer-forming liquid as the material for the formwork, the shape immediately after the application of the polymer layer-forming liquid can be stabilized, and the entrainment of foam can be prevented. Furthermore, a stable liquid level that is resistant to external disturbances such as wind and temperature unevenness during drying can be fixed. On the other hand, in a case where the wettability of the material forming the formwork with the polymer layer-forming liquid is poor, the liquid level of the coating liquid is a liquid level having a high curvature, even a slight shape unevenness causes a large difference in surface tension, the shape of the coating liquid is lost, and the coating liquid is repelled from the formwork, which is not preferable. In order to enhance wettability, adding a material which makes the raw material of the formwork hydrophilic and has a surface-active ability, such as a protein, to the polymer layer-forming liquid, is also an effective unit. Regarding the installation of the formwork 14, the formwork 14 may be installed from the drug solution charging step or may be installed before the polymer layer-forming liquid supplying step.

The height of the formwork 14 is preferably from 10 µm to 5000 µm. In order to fix the polymer layer-forming liquid 24 at the level difference part, it is necessary to apply the polymer layer-forming liquid 24 at a height higher than or equal to that of the formwork 14 and in an area larger than or equal to that of the formwork 14, and therefore, the amount of the polymer layer-forming liquid to be used can be suppressed by adjusting the height of the formwork 14 within this range. Therefore, the drying time can be shortened. In a case where the height of the formwork 14 is lower than 10 µm, the polymer layer-forming liquid 24 is not fixed at the formwork 14, the mold 13 repels the polymer layer-forming liquid, and the sheet part 116 is not formed, so that a micro-needle array cannot be manufactured.

In the polymer layer-forming liquid supplying step, the thickness of the polymer layer-forming liquid at the time of application is preferably higher than or equal to the height of the formwork 14 from the region 16 where the needle-shaped depressions 15 of the mold 13 are formed, and the thickness is preferably 5000 µm or less. It is because in order to fix the polymer layer-forming liquid at the position of the formwork 14, it is necessary to make the thickness of the polymer layer-forming liquid to be higher than or equal to the height of the formwork 14, and in a case where the coating thickness of the polymer layer-forming liquid after application is more than 5000 µm, it takes time to dry. Furthermore, in order to stably fix the polymer layer to the formwork while making the overall liquid level thin in order to reduce a drying load and to lower the manufacturing cost, a film thickness distribution in which the film thickness in the vicinity of the formwork is made thick and the film thickness in the vicinity of the center is made thin, may be adopted.

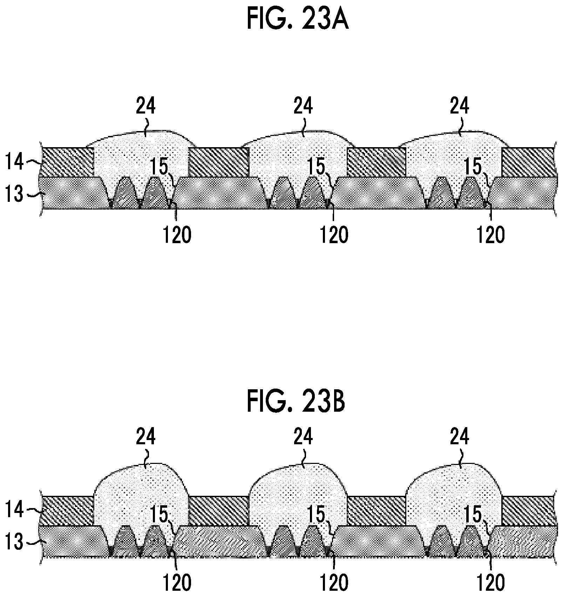

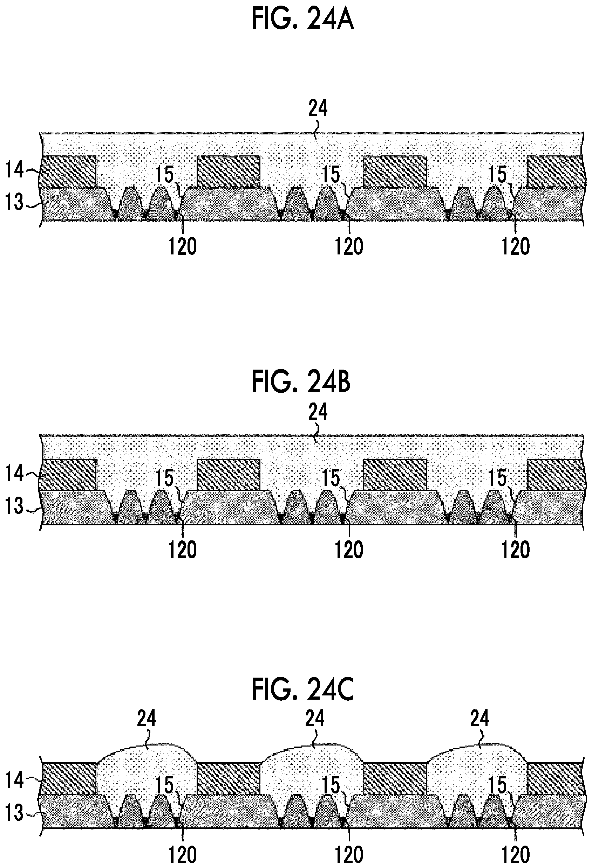

As a method of applying the polymer layer-forming liquid 24 to the mold 13, as shown in Figs. 23A and 23B, the polymer layer-forming liquid 24 may be applied on each of the needle-shaped depressions 15 having the periphery surrounded by the formwork 14, or as shown in Figs. 24A, 24B, and 24C, the polymer layer-forming liquid 24 may be applied over the entire surface of the mold 13 by covering the formwork 14. As shown in Fig. 23A, in a case where the polymer layer-forming liquid 24 is applied on each of the formworks 14, after the application, the polymer layer-forming liquid can be fixed by the formworks 14 as shown in Fig. 23B. Regarding a method of applying each of the formworks 14, the application can be carried out by methods such as intermittent stripe coating using a slit coater, a dispenser, inkjetting, letterpress printing, lithographic printing, and screen printing.

As shown in Fig. 24A, in the case of coating the entire surface of the mold 13, in a case where the amount of the polymer layer-forming liquid is large, the polymer layer-forming liquid drying step is carried out while the polymer layer-forming liquid 24 is uniformly applied, as shown in Fig. 24B. Even in this case, a polymer layer 122 can be stably formed in the subsequent polymer layer-forming liquid drying step. In a case where the amount of the polymer layer-forming liquid 24 is small, as shown in Fig. 24C, the polymer layer-forming liquid 24 contracts toward the inner side of the formwork 14 (region where the needle-shaped depressions 15 are formed) and is fixed by the formwork 14, manufacture of the micro-needle array with a stable shape can be carried out. Regarding a method of uniformly applying the liquid, general coating methods such as slit coating, slide coating, blade coating, bar coating, roll coating, gravure coating, dip coating, and spray coating can be used.

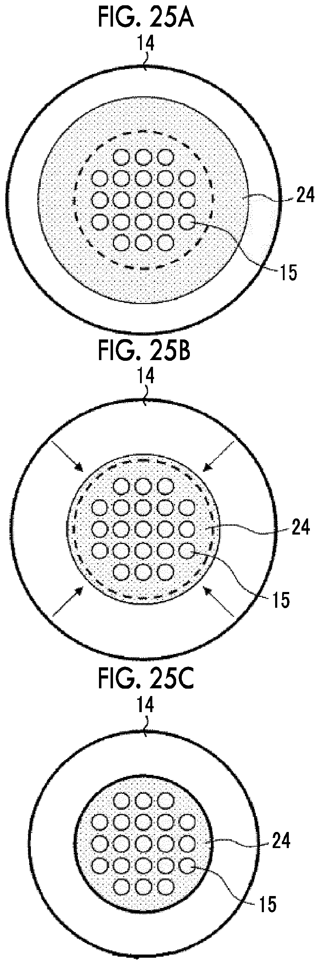

Figs. 25A to 27C are diagrams for explaining the contraction of the polymer layer-forming liquid according to the shape of the formwork. Figs. 25A, 25B, and 25C are diagrams showing that the polymer layer-forming liquid 24 is applied into a circular shape, and the polymer layer-forming liquid 24 is applied using a circular-shaped formwork 14 (Fig. 25A). As shown in Fig. 25B, since the polymer layer-forming liquid 24 contracts isotropically, the polymer layer-forming liquid 24 applied on a wider area than the formwork 14 can be fixed at the position of the level difference part formed by the formwork 14 (Fig.25C).

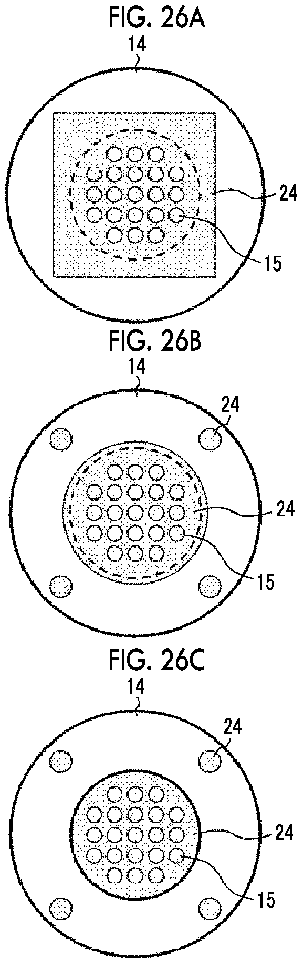

Figs. 26A, 26B, and 26C are diagrams in which the polymer layer-forming liquid 24 is applied into a quadrangular shape using a circular-shaped formwork 14 (Fig. 26A). Even in a case where the polymer layer-forming liquid 24 is applied into a quadrangular shape, the polymer layer-forming liquid 24 isotropically contracts toward the level difference part of the circular-shaped formwork 14 (Fig. 26B). Although there is a cissing residue on the formwork 14, the polymer layer-forming liquid 24 can be fixed at the position of the level difference part formed by the formwork 14 (Fig. 26C).

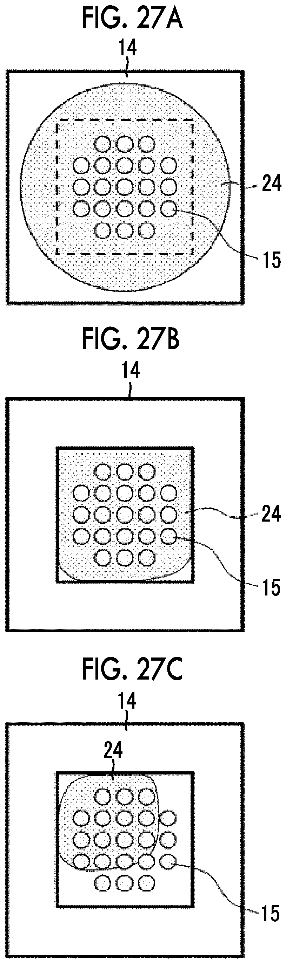

Figs. 27A, 27B, and 27C are diagrams showing that the polymer layer-forming liquid 24 is applied in a circular shape using a quadrangular-shaped formwork 14 (Fig. 27A). In a case where the shape of the formwork 14 is a quadrangular shape, in a case where the polymer layer-forming liquid 24 contracts isotropically, the polymer layer-forming liquid 24 can be fixed at the quadrangular-shaped corners of the formwork 14, and the polymer layer-forming liquid 24 may be wetted and fall off from the formwork 14 (Fig.27B). The polymer layer-forming liquid 24 that has been wetted and fallen off from the formwork 14 further contracts on the mold, a polymer layer is not formed in the portion where the needle-shaped depressions 15 are formed, and the micro-needle array may not be stably formed (Fig.27C).

As shown in Figs. 24A, 24B, and 24C, in the case where the polymer layer-forming liquid is uniformly applied on the mold, the polymer layer-forming liquid supplying step can be carried out, even if the shape of the formwork 14 is a quadrangular shape, without having the polymer layer-forming liquid wetted and fallen off within the formwork. In order to fix the polymer layer-forming liquid in the level difference part formed by the formwork, it is preferable that all the shapes around the region where the needle-shaped depressions are formed by providing the formwork are made into a polygon such as a hexagon or a higher polygon, in which all corners are formed at an angle of 120° or more as viewed from the top face, and more preferably a polygon such as a regular hexagon or a higher polygon, or a circle. By making the shape of the level difference part of the formwork into the above-described shape, at the time of applying the polymer layer-forming liquid, the contractile force due to the surface tension of the polymer layer-forming liquid acting on the level difference part provided in the mold can be made uniform. In the "regular polygon", it is preferable that the respective sides constituting the polygon are equal; however, changes can be made to the extent that the effect of the invention is obtained.

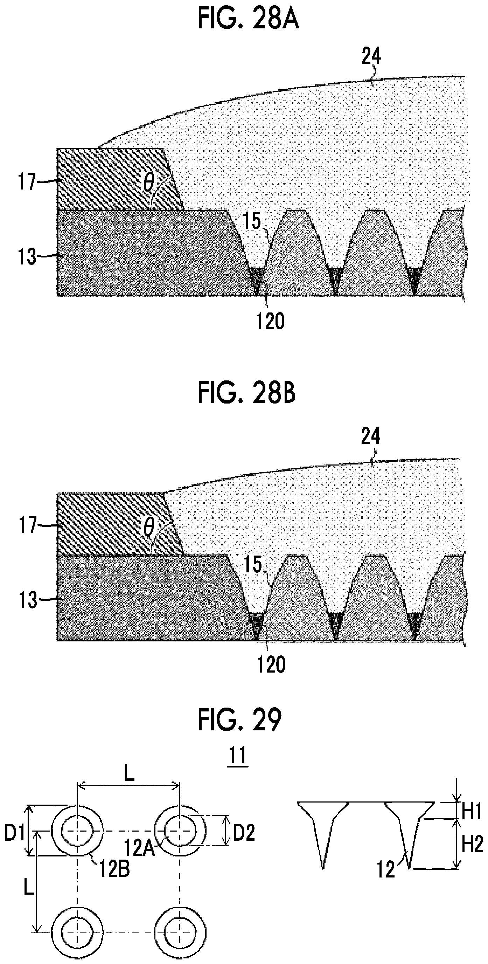

Figs. 28A and 28B are diagrams illustrating a polymer layer-forming liquid supplying step using a formwork 17 having a shape tapered in directions spreading upward from the side of the region where the needle-shaped depressions of the mold 13 are formed. Even in the case of the formwork 17 having a tapered shape, the polymer layer-forming liquid 24 is applied in an area larger than or equal to the region on the upper side of the formwork 17 (Fig.28A), and then is contracted by surface tension, and thereby the polymer layer-forming liquid 24 can be fixed at the level difference part formed by the formwork 17 (Fig.28B).

Furthermore, by forming the formwork 17 into a tapered shape in directions spreading upward in the vertical direction, there is an effect of removing foam that has mixed into the polymer layer-forming liquid 24. By removing the foam mixed into the polymer layer-forming liquid 24, loss of the needle-shaped protrusions during the detaching step and damage of the needle-shaped protrusions during tapping can be prevented.

As the taper angle θ of the formwork 17, it is preferable to adjust the angle formed with the mold 13 to be from 45° to 75°.

### (Polymer layer-forming liquid drying step)

Returning to Figs. 20A to 20D, after the polymer layer-forming liquid supplying step, the polymer layer-forming liquid 24 is dried and solidified as shown in Fig. 20D, so as to form the polymer layer 122 on the drug layer 120. A polymer sheet 1 having the drug layer 120 and the polymer layer 122 is manufactured.

In order to stably fix the polymer layer to the formwork, it is effective to quickly dry the polymer layer-forming liquid at the level difference part. It is preferable that the level difference part is dried and solidified by using a dry air flowing perpendicularly to the mold, increasing the wind speed, and using a high temperature and low humidity dry air.

In the polymer layer-forming liquid drying step, as the polymer layer-forming liquid 24 is dried, the volume is reduced. In a case where the polymer layer-forming liquid 24 is closely adhered to the mold 13 during drying, volume reduction occurs in the film thickness direction of the sheet, and the film thickness is thinned.

In a case where the polymer layer-forming liquid 24 is detached from the mold 13 during drying, the polymer sheet 1 may be distorted or curled because it contracts in the planar direction as well. In a case where the polymer sheet 1 is detached from the mold 13 in a state in which the polymer layer-forming liquid 24 in the needle-shaped depressions 15 has not been sufficiently dried, the shape of the needle-shaped protrusions of the polymer sheet 1 is likely to be subjected to defects such as breaking or bending. Therefore, it is preferable that the polymer sheet 1 does not detach from the mold 13 during drying. Furthermore, in order to suppress curling, a layer that contracts to the same extent as the front surface having the needle-shaped protrusions may be formed on the back surface (surface on the opposite side of the surface where the needle-shaped protrusions are formed) of the polymer sheet 1. For example, the same polymer solution as that on the front surface side is applied on the back surface side, and a layer is formed so as to obtain a film thickness with which the effect of suppressing curling has been checked in advance.

### (Detaching step)

The method for detaching the polymer sheet 1 from the mold 13 is not limited. It is desirable that the needle-shaped protrusions do not bend or break during detachment. Specifically, a sheet-like base material on which a pressure-sensitive adhesive layer having pressure-sensitive adhesiveness is formed is attached onto the polymer sheet 1, and then detachment can be performed so as to tear off the base material from an edge. A method of installing a sucker on the back surface of the polymer sheet 1 and pulling up the polymer sheet vertically while sucking the polymer sheet with air, can be applied. The micro-needle array 100 is manufactured by detaching the polymer sheet 1 from the mold 13.

### (Degassing step)

It is preferable to degas the drug solution 22 and/or the polymer layer-forming liquid 24 before the drug solution charging step and/or before the polymer layer-forming liquid supplying step. By degassing, the air bubbles included in the drug solution 22 and the polymer layer-forming liquid 24 can be removed before the drug solution and the polymer layer-forming liquid are charged into the needle-shaped depressions 15 of the mold 13. For example, in the degassing step, air bubbles having a diameter of 100 µm to several mm are removed.

Regarding a degassing method, for example, (1) a method of exposing the drug solution 22 to a reduced pressure environment for 1 to 15 minutes, (2) a method of ultrasonically vibrating the container accommodating the drug solution 22 for 5 to 10 minutes, (3) a method of ultrasonically vibrating the drug solution 22 while exposing the drug solution to a reduced pressure environment, and (4) a method of replacing dissolved gas with helium by sending helium gas into the drug solution 22, may be mentioned. The degassing methods (1) to (4) can also be applied to the polymer layer-forming liquid 24.

### (Pressing member)

The micro-needle array device according to the embodiments the invention comprises a pressing member that presses a surface of the sheet part, the surface being on the opposite side of the surface having the needle parts, at the time of tapping the needle parts.

It is preferable that the pressing member is installed at the center, or substantially the center, of the region where the needle parts of the micro-needle array exist. The phrase "the pressing member is installed at the center, or substantially the center, of the region where the needle parts of the micro-needle array exist" implies that in a case where the center of the region where the needle parts of the micro-needle array exist is designated as the center of a circle circumscribing the outer periphery of the needle arrangement, and the center of a pressing face (surface on which the pressing member comes into contact with the region where the needle parts of the micro-needle array exist) is designated as the center of a circle circumscribing the outer periphery of the pressing face, the distance between the two centers is within 20% of the diameter of a circle circumscribing the region where the needle parts of the micro-needle array exist.

According to the embodiments of the invention, the area A of the pressing face of the pressing member that presses the sheet part of the micro-needle array and the area B of the region where the needle parts of the micro-needle array exist satisfy the formula: 0.3 ≤ A/B ≤ 0.75. Thereby, a micro-needle array device whose needle parts can be sufficiently dissolved can be provided.

The area A and the area B preferably satisfy the formula: 0.40 ≤ A/B ≤ 0.75, more preferably the formula: 0.40 ≤ A/B ≤ 0.70, and even more preferably the formula: 0.40 ≤ A/B ≤ 0.60.

The area A of the pressing face is preferably from 30 mm² to 250 mm², more preferably from 40 mm² to 240 mm², and even more preferably from 50 mm² to 230 mm².

The area B of the region where the needle parts exist is preferably from 100 mm² to 400 mm², more preferably from 110 mm² to 350 mm², and even more preferably from 115 mm² to 320 mm².

The pressing face is preferably flat. The shape of the pressing face is not particularly limited; however, the shape can be circular or polygonal.

The material for the pressing member is not particularly limited; however, the material is preferably a material having a hardness sufficient for the micro-needle array to be pressed against the skin. As a material having a hardness sufficient for the micro-needle array to be pressed against the skin, a material that is not easily deformed by a load is preferable, a material having a tensile modulus of 500 MPa or more is more preferable, and a material having a tensile modulus of 1000 MPa or more is even more preferable. Specific examples of the material for a tapping device 70 include paper, paperboard, plastics, wood, glass, and metals. From the viewpoint of economic efficiency and ease of disposal, paper, paperboard, plastics, and wood are preferred as the material for the pressing member, and paper, paperboard, plastics, and wood having a tensile modulus of 500 MPa or more or 1000 MPa or more are more preferred. The pressing member can be produced by a known manufacturing technology such as, for example, compression molding, injection molding, forging, or casting, according to the material.

In the case of selecting paper or paperboard as the material for the pressing member, hard paper or a multilayer paperboard obtained by providing a coating of polyethylene, polypropylene, or the like on one side (or both sides) of hard paper, can be used. In the case of selecting a plastic as the material for the pressing member, polyethylene, polypropylene, polystyrene, polycarbonate, acrylic, polyethylene terephthalate (PET), and the like are preferred, and polypropylene, polystyrene, polycarbonate, acrylic, and polyethylene terephthalate (PET) are more preferred. Hard paper or a multilayer paperboard obtained by providing a coating of vinyl acetate, polyethylene, polypropylene, or the like on one side (or both sides) of hard paper, can be used.

In the micro-needle array device, it is preferable that the pressing member and the sheet part are adhered to each other.

### (As to tapping conditions)

Regarding the conditions at the time of tapping the micro-needle array into the skin, for example, tapping to the skin can be achieved with an energy quantity of 0.1 J to 1.0 J, preferably 0.1 J to 0.5 J, and more preferably 0.1 J to 0.4 J. In a case where the micro-needle array is tapped into the skin by hand, the micro-needle array is generally tapped into the skin with an energy quantity of 0.1 to 0.2 J. 1 J is 1 N·m or 1 m²·kg·s⁻².

After tapping under the above-described conditions, it is preferable to fix the micro-needle array on the skin with a force of, for example, 1 N (Newton) to 10 N, preferably 1.5 N to 8 N, and more preferably 2 N to 7 N, for 1 to 30 minutes.

According to the present invention, there is provided a method for administering a micro-needle array. The method comprises a step of tapping a micro-needle array device into the skin with an energy quantity of 0.1 to 1.0 J (preferably 0.1 J to 0.5 J, and more preferably 0.1 J to 0.4 J), the micro-needle array device including a micro-needle array having a sheet part and a plurality of needle parts existing on a top face of the sheet part; a pressing member for pressing a surface of the sheet part, the surface being on an opposite side of the surface having the needle parts, in which the sheet part has a thickness of 0.1 to 0.6 mm, and an area A of a pressing face of the pressing member for pressing the sheet part of the micro-needle array and an area B of a region where the needle parts of the micro-needle array exist, satisfy the formula: 0.3 ≤ A/B ≤ 0.75, and a step of fixing the micro-needle array on the skin for 1 to 30 minutes with a force of 1 N to 10 N (preferably 1.5 N to 8 N, and more preferably 2 N to 7 N).

### (Administration method)

As an example, the pressing member can be used in a state of being provided in an applicator, which will be described later. The pressing member can also be used, as another example, together with a container for finger tapping, which will be described later.

### <Applicator>

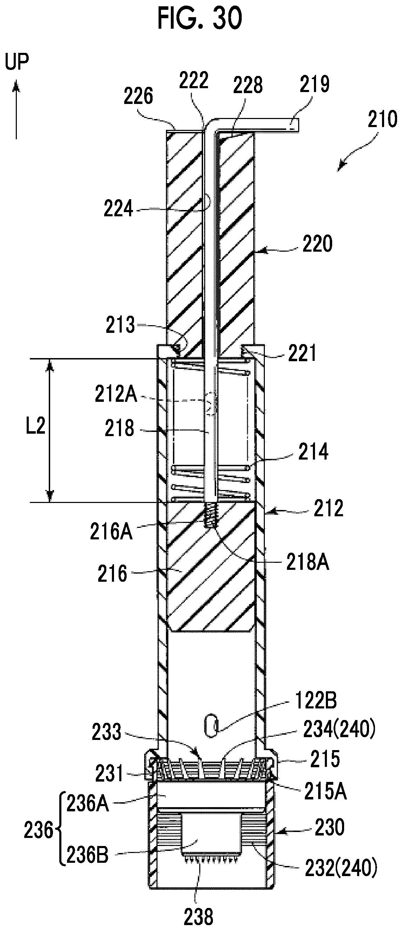

As shown in Fig. 30 to Fig. 32, the applicator 210 comprises a cylindrical-shaped main body 212; a substantially cylindrical-shaped temporary fixing member 220 that is attachably and detachably attached by being screwed to the upper end of the main body 212; and a cylindrical-shaped holder 230 that can be attachably and detachably attached by being fitted to the lower end of the main body 212. It is preferable that the main body 212 (including a first piston 216 that will be described later), the temporary fixing member 220, and the holder 230 (including a second piston 236 that will be described later) are made of a lightweight resin having a low friction coefficient (for example, made of polytetrafluoroethylene).

Inside the main body 212, a coil spring 214 as an urging member and the columnar first piston 216 are provided in this order from the top, and the first piston 216 is provided so as to strike the lower part of the main body 212 by the urging force of the coil spring 214. The outer diameter of the first piston 216 is adjusted to be almost the same as the inner diameter of the main body 212 (inner diameter of a main body 212 - outer diameter of a first piston 216 = 0.02 mm to 0.06 mm), and the outer diameter of a coil spring 214 is formed to be slightly smaller than the inner diameter of the main body 12.

Furthermore, a lower end of a lever member 218 is attached to the axial center at the upper end of the first piston 216. That is, a screw hole part 216A as a female screw part is formed at the axial center at the upper end of the first piston 216, and a male screw part 218A is formed at the lower end of the lever member 218.

The male screw part 218A of the lever member 218 is screwed into the screw hole part 216A of the first piston 216, so that the first piston 216 is integrally attached to the lower end of the lever member 218. It is configured such that this lever member 218 pulls up the first piston 216 toward the upper side of the main body 212 against the urging force of the coil spring 214.

As shown in Fig. 30, on the peripheral wall of the main body 212, two intake and exhaust ports 212A and 212B, through which air is taken into the main body 212 concomitantly with pulling up of the first piston 216, and the air present inside the main body 212 is discharged concomitantly with striking of the first piston 216, are formed to be vertically separated from each other.

As shown in Fig. 30 to Fig. 32, the temporary fixing member 220 temporarily fixes the lever member 218 in a state in which the first piston 216 is pulled up against the urging force of the coil spring 214. The upper end of the lever member 218 is composed of an operation unit 219 bent at a right angle with respect to the axial direction of the main body 212. On the peripheral wall of the temporary fixing member 220, a slit part 224 that is in communication with a through-hole 222 formed at the axial center to penetrate through the lever member 218 except for the operation unit 219, and penetrates through the operation unit 219, is formed along the axial direction.

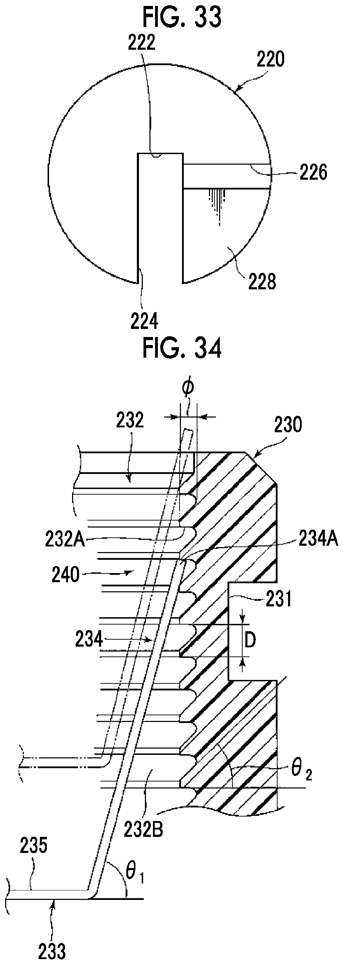

As shown in Fig. 33, at a position on the upper end face of the temporary fixing member 220 and appropriately 90 degrees away from the slit part 224 in the circumferential direction, a groove part 226 having an approximately "V"-shaped cross-section or a semicircular-shaped cross-section for retaining the operation unit 219 is formed so as to extend in the radial direction. A slope 228 is formed on the upper end ace of the temporary fixing member 220 and between the groove part 226 and the slit part 224, as a downwardly inclined surface that guides the operation unit 219 deviated from the groove part 226 to the slit part 224. In Fig. 30 to Fig. 32, the slope 228 is formed on one side, and a groove part 226 is formed on both sides.

As shown in Fig. 30 to Fig. 32, a male screw part 221 having a through-hole 222 as a rotation center is integrally formed at the lower end of the temporary fixing member 220, and a female screw part 213 into which the male screw part 221 is screwed is formed at the upper end of the main body 212. That is, the temporary fixing member 220 is attached by screwing such that the member can be easily removed from the main body 212, and is formed to be replaceable with a member having a different axial length.

As shown in Fig. 30 to Fig. 32, a diameter-expanded part 215 is formed at the lower end of the main body 212, and the upper end of the holder 230 is attachably and detachably fitted on the inner side in the radial direction of the diameter-expanded part 215. That is, a claw part 215A is formed along the circumferential direction at the lower end of the inner peripheral surface of the diameter-expanded part 215, and a groove part 231 into which the claw part 215A fits is formed along the circumferential direction at the upper end on the outer peripheral surface of the holder 230 (see also Fig. 34).

On the inner peripheral surface of the holder 230 (specifically, a region from the upper end face of the holder 230 to about 2/3 of the total length), a plurality of annular protrusions 232 as a locked part along the circumferential direction are formed side by side in the axial direction. Then, as shown in Fig. 34, the underside 232A as a stopper surface of the annular protrusions 232 is formed as a flat surface substantially facing the axial direction (preferably, the axial direction is the normal direction), and the top face 232B of the annular protrusions 232 is a downwardly inclined surface facing toward an axial center side (inward in the radial direction).

As shown in Fig. 30 to Fig. 32, a second piston 236 is provided inside the holder 230. The second piston 236 is formed into a two-stage columnar shape in which the upper side is a large diameter part 236A having an outer diameter that is almost the same as the inner diameter of the holder 230, and the lower side is a small diameter part 236B having an outer diameter that is smaller than the large diameter part 236A. Then, on the top face of the large diameter part 236A of the second piston 236, a claw-shaped member 233 made of a metal (for example, stainless steel) and having a claw part 234 as a locking part constituting the regulation unit 240 together with the annular protrusions 232 is provided.

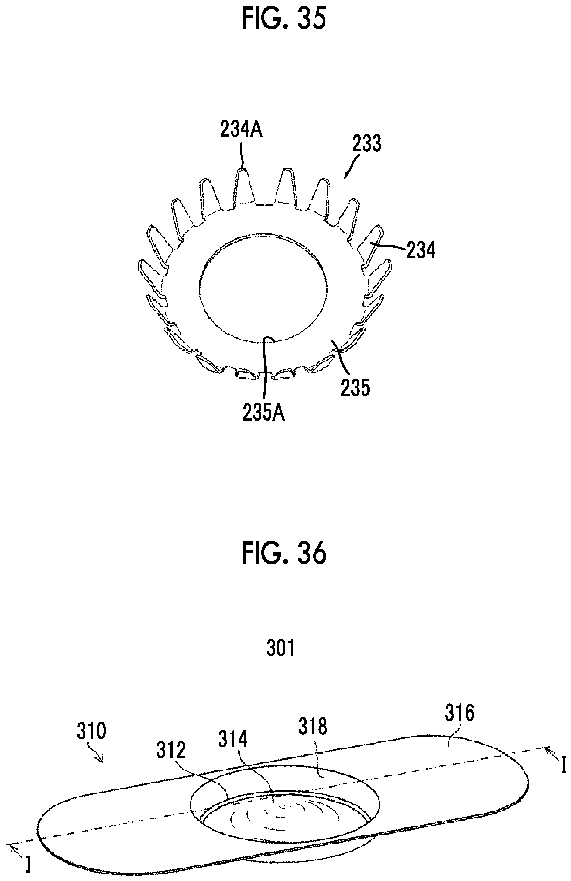

As shown in Fig. 35, the claw-shaped member 233 has a plurality (three or more, for example, 18) of the claw parts 234 that are integrally erected diagonally upward at equal intervals from an outer peripheral edge part of a flat plate ring-shaped base part 235. At least the tip 234A of each claw part 234 is elastically deformable toward the axial center side by being pushed toward the axial center side (inward in the radial direction) by the annular protrusions 232, concomitantly with the downward movement of the second piston 236.

As shown in Fig. 34, as the tip 234A of this claw part 234 hits the underside 232A of the annular protrusions 232 from below (in a direction opposite to the striking direction of the second piston 236), the upward rebound (movement) of the second piston 236 after hitting the skin is regulated. Therefore, it is preferable that the tip 234A of the claw part 234 is made into a flat surface so as to appropriately hit the underside 232A of the annular protrusions 232 (see Fig. 35).

As shown in Fig. 34, the standing angle θ1 of the claw part 234 with respect to the base part 235 is set to, for example, θ1 = 76 degrees. The inclination angle θ2 of the top face 232B with respect to the underside 232A of the annular protrusions 232 is set to, for example, θ2 = 45 degrees. Furthermore, the amount of projection ϕ inward in the radial direction of the annular protrusions 232 is, for example, ϕ = 0.25 mm, and the distance D in the axial direction of the annular protrusions 232 is, for example, D = 0.4 mm.

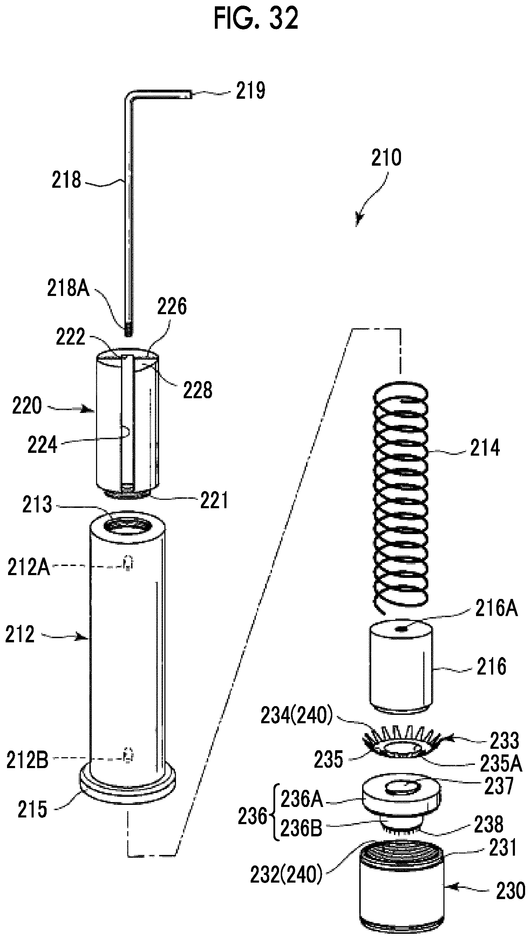

At the center of the top face of the large diameter part 236A of the second piston 236, a circular protrusion 237 (see Fig. 32) having the same outer diameter as the inner diameter of a circular through-hole 235A formed at the axial center of the base part 235 is protruding. Therefore, the base part 235 is joined to the top face of the second piston 236 with an adhesive or the like, in a state in which the through-hole 235A of the base part 235 is fitted to the protrusion 237 of the second piston 236. That is, the claw-shaped member 233 is attached, while being centered, to the top face of the second piston 236.

A micro-needle array is attached to the underside (surface facing the skin) of the small diameter part 236B of the second piston 236.

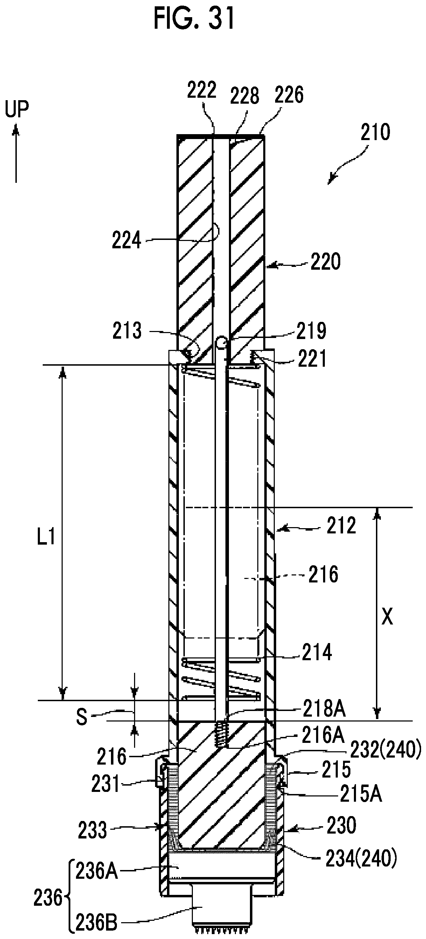

As shown in Fig. 30 and Fig. 31, in a case where the free length of the coil spring 214 is designated as L1; the total length of the coil spring 214 upon being compressed is designated as L2; and the amount of movement of the first piston 216 is designated as X, the formula: L1 < L2 + X should be satisfied. That is, as shown in Fig. 31, after the first piston 216 is struck, the upper end face of the first piston 216 is configured to be separated from the lower end face of the coil spring 214, and it is configured such that a gap S is formed between the lower end face of the coil spring 214 and the upper end face of the first piston 216.

### <Container for tapping by finger>

As shown in Fig. 36, a micro-needle array unit 301 comprises a container 310. The container 310 comprises an accommodation part 312 for accommodating the micro-needle array; a deformable part 314 integrated with the accommodation part 312; and a flange part 316 integrated with the accommodation part 312 and bent by a bendable part 318.

The accommodation part 312 and the deformable part 314 of the container 310 have a circular shape as viewed in a plan view. The flange part 316 of the container 310 has a race track shape (a shape combining two semicircles and straight lines) as viewed in a plan view. However, the shapes of the accommodation part 312, the deformable part 314, and the flange part 316 are not limited. The flange part 316 may be provided on the entire periphery of the accommodation part 312. The entire periphery means that the flange part 316 surrounds the whole circumference of the accommodation part 312. It is not necessary to provide the flange part 316 along the entire periphery of the accommodation part 312. It is preferable that the flange part 316 has an adhesive on the surface that comes into contact with the skin. The container 310 is stuck to the skin by the adhesive of the flange part 316. Even in a case where the flange part 316 does not have an adhesive, the container 310 is stuck to the skin by an adhesive applied on the skin. Furthermore, the container 310 is stuck to the skin by sticking another member (medical tape) or the like from above the container 310.

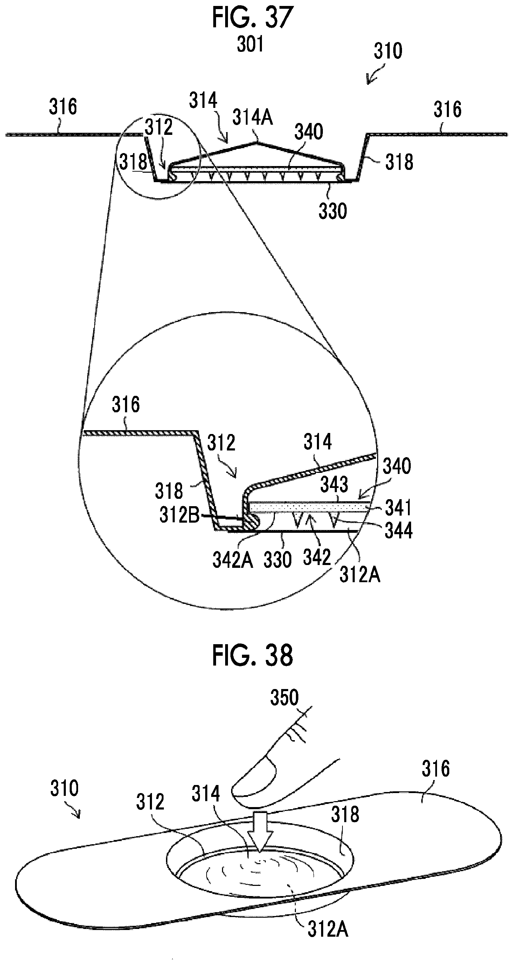

As shown in Fig. 37, the accommodation part 312 has an internal space defined by the inner walls, and an opening 312A. The opening 312A of the accommodation part 312 is sealed by a lid 330. The accommodation part 312 includes a protruding part 312B that is disposed on the inner wall and protrudes into the internal space.

The deformable part 314 is disposed on the opposite side of the opening 312A and is integrated with the accommodation part 312. The deformable part 314 has, for example, a convex shape having an apex part 314A separated from the micro-needle array 340. The apex part 314A of the deformable part 314 means a portion of the deformable part 314, which is most distantly separated from the micro-needle array 340, and the convex shape means that the apex part 314A is not located in the internal space of the accommodation part 312. The deformable part 314 can have a plurality of the apex parts 314A. The term "being integrated" means a state in which the accommodation part 312 and the deformable part 314 are connected to each other.

In a case where the accommodation part 312 and the deformable part 314 are integrated, it can be realized by separately molding the accommodation part 312 and the deformable part 314, fitting the accommodation part 312 with the deformable part 314, and then welding. In a case where the accommodation part 312 and the deformable part 314 are integrated, it may be either achieved before accommodating, or after accommodating, the micro-needle array 340 in the accommodation part 312. In a case where the accommodation part 312 and the deformable part 314 are integrated, it can be realized by integrally molding the accommodation part 312 and the deformable part 314. However, the method is not limited to these methods.

The deformable part 314 can have a gimlet shape or may have a conical shape. The deformable part 314 may have an internal space and can be in communication with the internal space of the deformable part 314 and the internal space of the accommodation part 312. The accommodation part 312 has a structure closed by the deformable part 314 on the opposite side of the opening 312A. The gimlet shape includes a conical shape or a pyramid shape and includes a frustum shape.

The flange part 316 is integrated with the accommodation part 312 and comes into contact with the skin. The flange part 316 extends outward from the position of the opening 312A of the accommodation part 312 and is further bent to the side of the deformable part 314 by a bendable part 318. The flange part 316 is disposed at a position beyond the apex part 314A of the deformable part 314 with reference to the opening 312A of the accommodation part 312. The flange part 316 is formed so as to be parallel to the sheet part of the micro-needle array 340. The term parallel includes being parallel and being substantially parallel. The shape of the flange part 316 is not particularly limited as long as it can come into contact with the skin. In a case where the accommodation part 312 and the flange part 316 are integrated, a method similar to the case of integrating the accommodation part 312 and the deformable part 314 can be applied.

As shown in Fig. 38, the container 310 is positioned on the skin. The opening 312A of the accommodation part 312 is positioned toward the skin, and the needle parts of the micro-needle array are directed toward the skin. An external force in the direction of the opening 312A is applied to the deformable part 314 by a finger 350.

The present invention will be described in more detail by way of the following Examples; however, the present invention is not intended to be limited to the Examples. Examples

### <Production of mold>

A precursor plate 11 was produced by grinding, on the surface of a smooth Ni plate that measured 40 mm on each side, protrusions 12 having a needle-shaped structure in which a cone 12A having a diameter D2 of 380 µm and a height H1 of 910 µm was formed on a truncated cone 12B having a bottom face with a diameter D1 of 800 µm and a height H2 of 210 µm, as shown in Fig. 29. On this precursor plate 11, a film having a thickness of 1070 µm was formed using a silicone rubber (SILASTIC-MDX4-4210 manufactured by Dow Corning Corporation), and the silicone rubber film was heat-cured while having 50 µm of the cone tip part of the precursor plate 11 protruded from the film surface, and was detached. Thereby, an inversion product of silicone rubber having through-holes with a diameter of about 30 µm was produced. This silicone rubber inversion product processed by cutting off the outside of a flat surface part that measured 30 mm on each side, in which needle-shaped depressions two-dimensionally arranged in an array of 10 columns×10 rows were formed at the central portion, was used as a mold. The side of the needle-shaped depressions with wider openings was designated as the front surface of the mold, and the surface having through-holes (air vent holes) having a diameter of 30 µm was designated as the back surface of the mold.

### <Preparation of polymer solution (polymer layer-forming liquid)>

Chondroitin sulfate (manufactured by Maruha Nichiro Corporation) was dissolved in water and was prepared into a 40 mass% aqueous solution, and this was used as a polymer layer-forming liquid. After the liquid was prepared, it was exposed to a reduced pressure environment at 3 kPa for 4 minutes so as to be sufficiently degassed.

### <Polymer layer-forming liquid supplying step and polymer layer-forming liquid drying step>

The following steps were carried out in an environment at a temperature of 23°C and a relative humidity of 35%RH.

A gas-permeable film (POREFLON FP-010 manufactured by Sumitomo Electric Industries, Ltd.) that measured 15 mm on each side was placed on a horizontal suction base, and the mold was installed on the film such that the front surface was facing up. The gas-permeable film and the mold were fixed to the suction base by reducing the pressure from the direction of a mold back surface at a suction pressure of -90 kPa as a gauge pressure.

A stainless steel (SUS304) formwork was placed on the mold, and the polymer layer-forming liquid was directly applied thereon. The application of the polymer layer-forming liquid was carried out on the formwork in a region having a radius larger by 1 mm than that of the formwork. The formwork size is the diameter of the formwork, and the liquid level height is the coating thickness of the polymer layer-forming liquid and represents the height from a mold front surface to the liquid level at the level difference part formed by installing the formwork. The experiment was conducted by using a formwork size of 17 mm or 25 mm, and setting the number of needle-shaped depressions to 109 or 292, respectively. The polymer layer-forming liquid was directly applied by setting the height of the formwork to 1000 µm.

Based on the description given above, a micro-needle array having a needle part area of 122 mm² (number of needles: 109 needles) (thickness of the support was 0.35 mm) and a micro-needle array having a needle part area of 315 mm² (number of needles: 292 needles) (thickness of the support was 0.35 mm) were produced. Furthermore, micro-needle arrays having a thickness of the support of 0.5 mm, 0.65 mm, or 0.8 mm were produced by adhering a polyethylene terephthalate plate (PET plate) having an appropriate thickness to the above-described micro-needle array having a thickness of the support of 0.35 mm using a double-sided tape.

The following tapping and dissolution test 1 and tapping and dissolution test 2 were carried out using these micro-needle arrays.

### <Tapping and dissolution test 1>

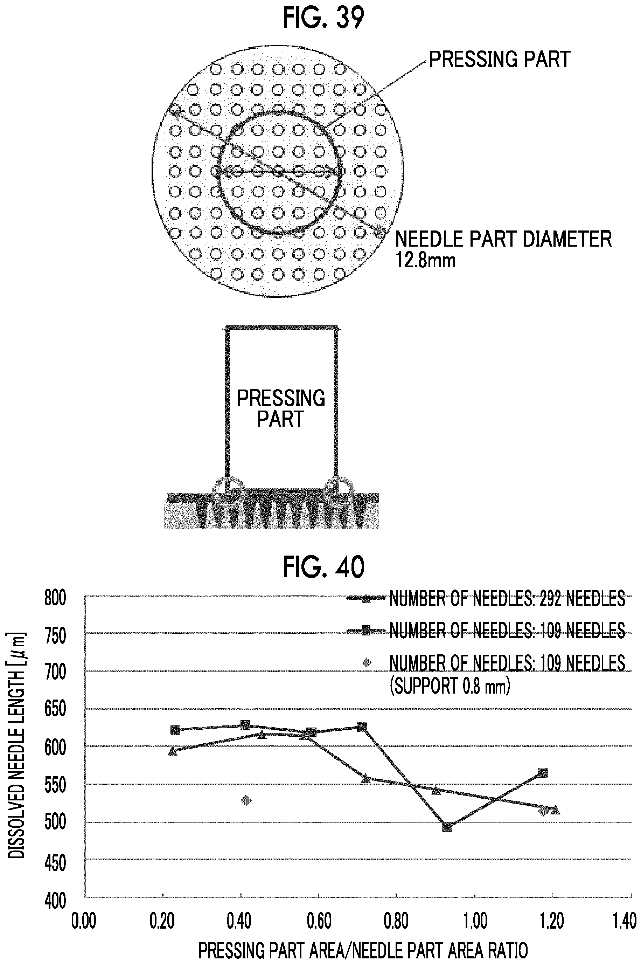

For domestic pigs (LWD strain), the dorsal surface was shaved under anesthesia, subsequently a micro-needle array was tapped with an energy quantity of 0.3 J using the applicator described in the above-described section <Applicator> in the present specification, and the micro-needle array was fixed with a force of 5 N for 10 minutes and then was detached. Regarding the applicator, applicators having the areas of the pressing part shown in Table 1 below were used. Fig. 39 shows the relationship between the needle part (the case with a needle part diameter of 12.8 mm and a needle part area of 122 mm²) and the pressing part. Force is applied to the outer periphery of the pressing part (site marked with a circle in the diagram below).

For excised pig skin (Yukatan Micro Pig Skin Set, Charles River Laboratories Japan, Inc.), the fat layer was removed, subsequently a micro-needle array was tapped with an energy quantity of 0.3 J using the applicator described in the above-described section <Applicator> in the present specification, and the micro-needle array was fixed with a force of 5 N for 10 minutes and then was detached. Regarding the applicator, applicators having the areas of the pressing part shown in Table 1 below were used.

**[Table 1]**

| Needle part area | Pressing part area | Pressing part area/needle part area |
|---|---|---|
| 122mm² Number of needles: 109 needles | 143mm² | 1.17 |
| | 113mm² | 0.93 |
| | 87mm² | 0.71 |
| | 71mm² | 0.58 |
| | 50mm² | 0.41 |
| | 28mm² | 0.23 |
| 315mm² Number of needles: 292 needles | 380mm² | 1.21 |
| | 284mm² | 0.90 |
| | 227mm² | 0.72 |
| | 177mm² | 0.56 |
| | 133mm² | 0.42 |
| | 71mm² | 0.22 |

### (Measurement of residual length of micro-needle array after tapping and dissolution)

The residual length of the needle-shaped protrusions on the micro-needle array after detachment was measured using a stereomicroscope.

### (Calculation of dissolved needle length of micro-needle array after tapping and dissolution)

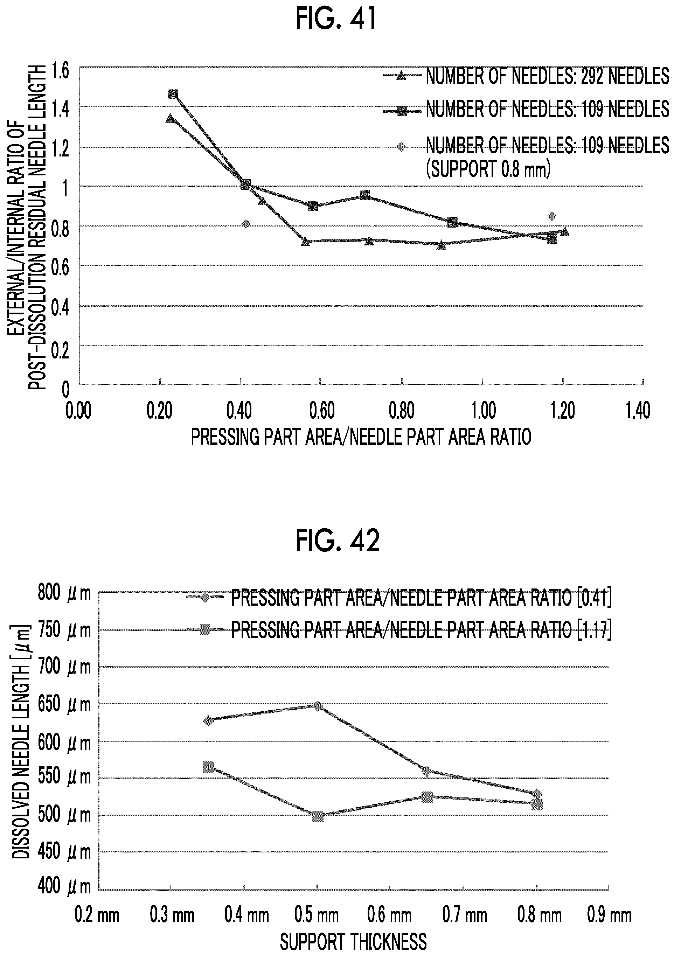

The dissolved needle length was calculated from the formula: needle length of the micro-needles before tapping and dissolution - residual length of the micro-needle array after tapping and dissolution, and was calculated by averaging with respect to the total number of needles. The results are shown in Fig. 40 and Fig. 42.

### (Calculation of external/internal ratio of post-dissolution residual needle length of micro-needle array after tapping and dissolution)

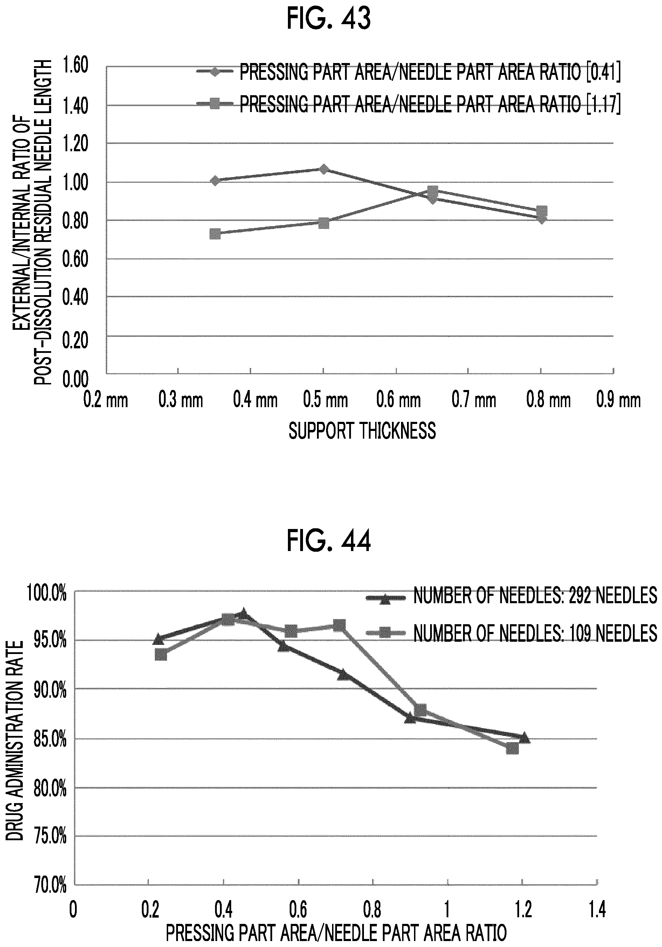

The ratio was calculated by the formula: average value of the residual length of the micro-needle array after tapping and dissolution on the outermost periphery/average value of the residual length of the micro-needle array after tapping and dissolution at a site other than the outermost periphery. The results are shown in Fig. 41 and Fig. 43.

### (Drug administration rate)

The drug-containing region in the needles is assumed as follows.

Needle tip to 430 µm: 60%, 430 µm to 500 µm: 30%, 500 µm to 575 µm: 8%, 75 µm to 830 µm: 2%

Here, 60%, 30%, 8% and 2% mean that 60%, 30%, 8% and 2% of the total amount of the drug are contained in the corresponding regions, respectively.

Based on the results shown in Fig. 40 and Fig. 41, the ratio of the amount of administered drug with respect to the total drug content was calculated from the amount of needle dissolved for each needle, and the ratio was averaged with respect to the total number of needles. The results are shown in Fig. 44.

From the results of Fig. 40 and Fig. 41, it was found that in a case where the area A of the pressing face of the pressing member and the area B of the region where the needle parts of the micro-needle array exist satisfy the formula: 0.3 ≤ A/B ≤ 0.75, the needles can be sufficiently dissolved.

From the result of Fig. 44, it is understood that in a case where the area A of the pressing face of the pressing member and the area B of the region where the needle parts of the micro-needle array exist satisfy the formula: 0.3 ≤ A/B ≤ 0.75, a high drug administration rate can be achieved.

From the results of Fig. 42 and Fig. 43, it was shown that the needles can be sufficiently dissolved in a case where the thickness of the sheet part is 0.1 to 0.6 mm.

### <Tapping and dissolution test 2>

A micro-needle array was tapped with a finger using the containers shown in Fig. 36, Fig. 37, and Fig. 38 instead of the applicator used in the tapping and dissolution test 1.

The pressing member is accommodated between the apex part 314A of the deformable part 314 and the micro-needle array 340, the deformable part 314 is pressed with a finger 350 or the like, and an external force in the direction of the opening 312A is applied. Thereby, the deformable part 314 is deformed, and by pressing the other surface of the micro-needle array 340, the micro-needle array 340 passes through the protruding part and is pushed out from the accommodation part 312 to the outside, the deformable part 314 maintains a deformed state, while the micro-needle array 340 is pressed against the skin and tapped into the skin.

The results of calculating the dissolved needle length of the micro-needle array after tapping and dissolution, and the external/internal ratio of the post-dissolution residual needle length of the micro-needle array after tapping and dissolution are shown below.

**[Table 2]**

| | |
|---|---|
| Number of needles | 109 needles |
| Needle part area | 122mm² |
| Pressing part area | 50mm² |
| Pressing part area/needle part area | 0.41 |
| Dissolved needle length | 641µm |
| External/internal ratio of post-dissolution residual needle length | 0.91 |

### Explanation of References

1: polymer sheet
11, 71, 81: precursor plate
12: protrusion
13, 73, 83: mold
14,17: formwork
15: needle-shaped depression
15A: inlet part
15B: tip depression
15C: through-hole
16: region
18: mold composite
19: gas-permeable sheet
20: base
22: drug solution
24: polymer layer-forming liquid
30: liquid feeding tank
32: piping
34: nozzle
34A: lip part
34B: opening
34C: inclined surface
36: liquid supply apparatus
48: drug solution charging apparatus
50, 54: axis driving unit
52: suction base
56: trestle
58: control system
60: displacement meter
74, 75, 84, 85: level difference part
92: coating unit
100: percutaneous absorption sheet
110: needle-shaped protrusion
112: needle part
112A: needle-shaped part
112B: body part
114: frustum part
116: sheet part
120: drug layer
122: polymer layer
210: applicator
212: main body
212A: intake and exhaust port
212B: intake and exhaust port
213: female screw part
214: coil spring (urging member)
215: Diameter-expanded part
215A: claw part
216: first piston
216A: screw hole part
218: lever member
218A: male screw part
219: operation unit
220: temporary fixing member
221: male screw part
222: through-hole
224: slit part
226: groove part
228: slope (inclined surface)
230: holder
231: groove part
232: annular protrusion
232A: underside
232B: top face
233: claw-shaped member
234: claw part (locking part)
234A: tip
235: base part
235A: through-hole
236: second piston
236A: large diameter part
236B: small diameter part
237: protrusion
240: regulation unit
θ1: angle
θ2: angle
ϕ: projection amount
D: interval
S: gap
301: micro-needle array unit
310: container
312: accommodation part
312A: opening
312B: protruding part
314: deformable part
314A: apex part
316: flange part
318: bendable part
330: lid
340: micro-needle array
341: sheet part
342: one surface
342A: outer peripheral surface
344: needle part
350: finger

## Claims

1. A micro-needle array device comprising:
a micro-needle array (340) having a sheet part (341) and a plurality of needle parts (344) existing on a top face of the sheet part; and
a pressing member for pressing a surface of the sheet part, the surface being on an opposite side of the surface having the needle parts,
wherein the sheet part has a thickness of 0.1 to 0.6 mm, and
an area A of a pressing face of the pressing member for pressing the sheet part of the micro-needle array and an area B of a region where the needle parts of the micro-needle array exist, satisfy the formula: 0.3 ≤ A/B ≤ 0.75.

2. The micro-needle array device according to claim 1, wherein the micro-needle array is formed of a water-soluble polymer.

3. The micro-needle array device according to claim 1 or 2, wherein the area A of the pressing face is from 30 mm² to 250 mm², and the area B of the region where the needle parts exist is from 100 mm² to 400 mm².

4. The micro-needle array device according to any one of claims 1 to 3, wherein the pressing face is a flat surface.

5. The micro-needle array device according to any one of claims 1 to 4, wherein the pressing face is circular-shaped.

6. The micro-needle array device according to any one of claims 1 to 4, wherein the pressing face is polygonal-shaped.

7. The micro-needle array device according to any one of claims 1 to 6, wherein the pressing member and the sheet part are adhered to each other.

## Patentansprüche

1. Mikronadelfeld-Vorrichtung, umfassend:
ein Mikronadelfeld (340) mit einem Flachstückteil (342) und einer Mehrzahl von Nadelteilen (344), die sich auf einer Oberseite des Flachstückteils befinden;
ein Anpresselement zum Anpressen einer Oberfläche des Flachstückteils, die sich auf einer abgewandten Seite von der mit den Nadelteilen bestückten Oberfläche befindet,
wobei das Flachstückteil eine Dicke von 0,1 bis 0,6 mm aufweist, und
eine Fläche A einer Anpressfläche des Anpresselements zum Anpressen des Flachstückteils des Mikronadelfelds und eine Fläche B einer Zone, in welcher die Nadelteile des Mikronadelfelds vorhanden sind, der Formel: 0,3 ≤ A/B ≤ 0,75 genügen.

2. Vorrichtung nach Anspruch 1, bei der das Mikronadelfeld durch ein wasserlösliches Polymer gebildet ist.

3. Vorrichtung nach Anspruch 1 oder 2, bei die Fläche A der Anpressfläche von 30 mm² bis 250 mm² beträgt und die Fläche B der Zone, in der die Nadelteile vorhanden sind, von 100 mm² bis 400 mm² groß ist.

4. Vorrichtung nach einem der Ansprüche 1 bis 3, bei der die Anpressfläche eine flache Fläche ist.

5. Vorrichtung nach einem der Ansprüche 1 bis 4, bei der die Anpressfläche kreisförmig ist.

6. Vorrichtung nach einem der Ansprüche 1 bis 4, bei der die Anpressfläche polygonalförmig ist.

7. Vorrichtung nach einem der Ansprüche 1 bis 6, bei der das Anpresselement und das Flachstückteil aneinander haften.

## Revendications

1. Dispositif à réseau de micro-aiguilles, comprenant :
un réseau de micro-aiguilles (340) présentant une partie de feuille (341) et une pluralité de parties d'aiguilles (344) existant sur une face supérieure de la partie de feuille, et
un élément de pression pour presser une surface de la partie de feuille, la surface étant sur un côté opposé de la surface présentant les parties d'aiguilles ;
dans lequel la partie de feuille présente une épaisseur allant de 0,1 à 0,6 mm, et
une zone A d'une face de pression de l'élément de pression pour presser la partie de feuille du réseau de micro-aiguilles, et une zone B, où existent les parties d'aiguilles du réseau de micro-aiguilles, satisfont la formule : 0,3 ≤ A/B ≤ 0,75.

2. Dispositif à réseau de micro-aiguilles selon la revendication 1, dans lequel le réseau de micro-aiguilles est formé d'un polymère soluble dans l'eau.

3. Dispositif à réseau de micro-aiguilles selon la revendication 1 ou 2, dans lequel la zone A de la face de pression s'étend de 30 mm² à 250 mm², et la zone B de la région, où existent les parties d'aiguilles, s'étend de 100 mm² à 400 mm².

4. Dispositif à réseau de micro-aiguilles selon l'une quelconque des revendications 1 à 3, dans lequel la face de pression est une surface plate.

5. Dispositif à réseau de micro-aiguilles selon l'une quelconque des revendications 1 à 4, dans lequel la face de pression est en forme de cercle.

6. Dispositif à réseau de micro-aiguilles selon l'une quelconque des revendications 1 à 4, dans lequel la face de pression est en forme de polygone.

7. Dispositif à réseau de micro-aiguilles selon l'une quelconque des revendications 1 à 6, dans lequel la face de pression et la partie de feuille sont collées l'une à l'autre.
